# EUROPEAN PATENT APPLICATION

(11) **EP 4 137 499 A1**
(43) Date of publication of application: **22.02.2023**
(21) Application number: 21191715.8
(22) Date of filing: 17.08.2021
(51) Int. Cl.: C07H 21/02, A61P 35/00, A61K 31/7048

(54) **7-SUBSTITUTED 7-DEAZAADENINE-CONTAINING 2,3 -CYCLIC DINUCLEOTIDES**

(71) Applicant: Ustav organicke chemie a biochemie AV CR, v.v.i., 166 10 Praha 6 (CZ)
(72) Inventor: Hocek, Michal, Praha 6 (CZ); Birkus, Gabriel, South Lake Tahoe (US); Perlikova, Pavla, Praha 9 (CZ); Vavrina, Zdenek, Kutna Hora (CZ); Novotna, Barbora, Praha 8 (CZ)
(74) Representative: Hartvichova, Katerina

(57) **Abstract**

Title of the invention: 7-Substituted 7-deazaadenine-containing 2',3'-cyclic dinucleotides.

The invention provides 7-substituted 7-deazaadenine-containing 2',3'-cyclic dinucleotides of general formula I, wherein R is selected from the group comprising C1-C5 alkyl; C6-C16 aryl, optionally substituted by at least one substituent selected from C6-C16 aryl or (C6-C16 aryloxy)C1-C5 alkyl; C4-C12 heteroaryl, comprising at least one O atom; C4-C12 heteroaryl, comprising at least one S atom, and pharmaceutically acceptable salts thereof. The compounds according to the invention show strong activation of protein STING (stimulator of interferon genes) and, consequently, stimulation of the signal transduction pathway that induces interferons and other cytokines/chemokines.

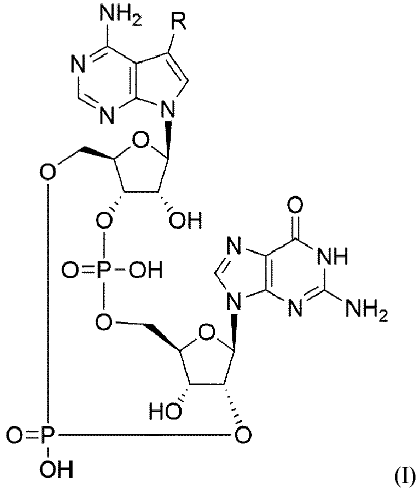

## Description

### Field of Art

The invention provides a new type of 2',3'-cyclic dinucleotides that may be useful in the treatments of diseases in which modulation of STING adaptor protein (Stimulator of Interferon Genes) is beneficial, for example, inflammation, allergic and autoimmune diseases, cancer, and viral infections such as chronic hepatitis B and human immunodeficiency virus, and in the preparation of immunogenic compositions or vaccine adjuvants.

### Background of Art

Stimulator of Interferon Genes (STING) protein plays crucial role in defence against pathogen infections, immune surveillance of tumor cells and maintaining the normal immune functions of the body (Barber, G. N., Nat. Rev. Immunol. 2015, 15, 760-770; Kumar, V., J. Leukoc. Biol. 2019, 106, 171-185). Development of potent human STING agonists is therefore a goal of research programs focusing on development of anti-viral and anti-cancer agents, on adjuvants in vaccines or on treatment of allergic or other inflammatory diseases. Among others, cyclic dinucleotides (CDNs) show activation of STING signalling and their analogs were designed in order to improve their potency and stability (Zhang, H. et al., J. Med. Chem. 2020, 63, 3785-3816).

7-Deazaadenine-containing CDNs have been previously prepared either by chemical synthesis or by enzymatic cyclization of corresponding nucleoside triphosphates. However, only derivatives bearing either unsubstituted 7-deazaadenine, 7-fluoro-7-deazaadenine or 7-cyano-7-deazaadenine (formula A; Rosenthal, K., et al., ChemBioChem 2020, 21, 3225-3228; Novotná B., et al., J. Med. Chem. 2019, 62, 10676-10690; WO2017161349A1, WO2018100558A2, WO2017027646A1) were published so far.

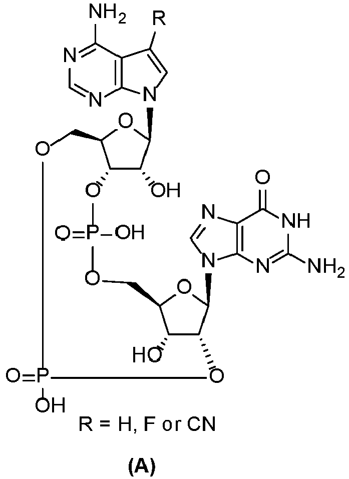

### Summary of the Invention

This invention provides novel 2',3'-cyclic dinucleotides (CDNs) of general formula I that bind to and activate protein STING and, consequently, stimulate the signal transduction pathway that induces interferons and other cytokines/chemokines. Such compounds may find utility as an anti-viral and anti-cancer agent, act as adjuvants in vaccines or may be used in the treatment of allergic or other inflammatory diseases.
The hydrocarbon substituent in position 7 of 7-deazaadenine makes these compounds significantly different from all previously prepared 7-deazapurine-containing CDNs of general formula A.
7-Substituted 7-deazaadenine-containing CDNs presented herein are a new class of compounds, which was not described previously. 7-Substituted 7-deazaadenine-containing CDNs mentioned above are a new and unique type of CDNs with a carbon substituent attached to the 7-deazaadenine nucleobase, which leads to a new type of interaction with STING protein providing enhanced stabilization of CDN-STING complex. Therefore, the activation of cGAS-STING signalling pathway by 7-substituted 7-deazaadenine-containing CDNs is stronger compared to that of other CDNs. Furthermore, thanks to increased lipophilicity, 7-substituted 7-deazaadenine-containing CDNs can penetrate cell membranes more easily which improves their performance in cell-based assays.

The object of the present invention is 7-substituted 7-deazaadenine-containing 2',3'-cyclic dinucleotide (CDN) of general formula I: 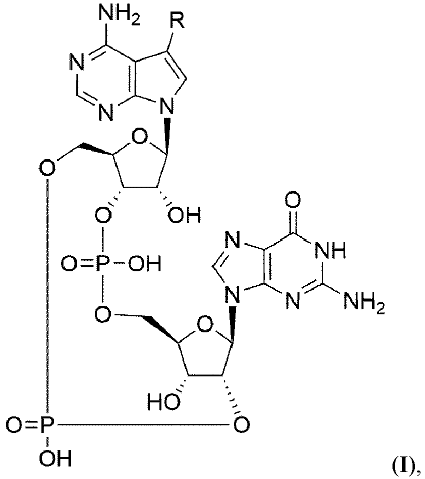 wherein
R is selected from the group comprising:
   - C1-C5 alkyl;
   - C6-C16 aryl, optionally substituted by at least one substituent selected from C6-C16 aryl or (C6-C16 aryloxy)C1-C5 alkyl;
   - C4-C12 heteroaryl, comprising at least one O atom (preferably comprising one O atom);
   - C4-C12 heteroaryl, comprising at least one S atom (preferably comprising one S atom);
and pharmaceutically acceptable salts thereof.

When compounds of formula **I** are optically active, formula **I** shall be understood as including individual optical isomers and mixtures of optical isomers, including racemic mixtures.

In some embodiments, R is C6-C16 aryl substituted by at least one substituent selected from C6-C16 aryl or (C6-C16 aryloxy)C1-C5 alkyl. Preferably, R is phenyl substituted by one substituent selected from C6-C16 aryl or (C6-C16 aryloxy)C1-C5 alkyl.

In one preferred embodiment, R is selected from the group comprising C1-C5 alkyl, phenyl, naphthalenyl, phenanthrenyl, furanyl, thiophenyl, benzofuranyl, benzothiophenyl, dibenzofuranyl, [1,1'-biphenyl]yl, (naphthalenyl)phenyl or [(naphthalenyloxy)methyl]phenyl.

More preferably, R is selected from the group comprising methyl, phenyl, naphthalen-1-yl, naphthalen-2-yl, phenanthren-9-yl, furan-2-yl, thiophen-2-yl, benzo[*b*]furan-2-yl, benzo[*b*]thiophen-2-yl, dibenzo[*b,d*]furan-4-yl, [1,1'-biphenyl]-4-yl, 4-(naphthalenyl)phenyl, 4-[(naphthalen-1-yloxy)methyl]phenyl.

As described herein and unless otherwise indicated, the individual substituents have the following meanings:
- alkyl is a linear or branched hydrocarbon chain containing the number of carbons indicated at each occurrence of the term. Examples of C1-C5 alkyl substituents include, but are not limited to, methyl, ethyl, propan-1-yl, propan-2-yl, butan-1-yl, butan-2-yl, 2-methylpropan-1-yl, 2-methylpropan-2-yl, pentan-1-yl, pentan-2-yl, pentan-3-yl, 2-methylbutan-2-yl, 3-methylbutan-2-yl, 3-methylbutan-1-yl, 2-methylbutan-1-yl, 1,1-dimethylpropan-1-yl;
- aryl is a hydrocarbon chain comprising at least one aromatic ring and containing the number of carbons indicated each occurrence of the term. The aryl may also contain more than one aromatic ring, then these rings may be condensed or non-fused. Aryl also encompasses multiple condensed ring systems in which at least one ring is aromatic and wherein the other rings may be aromatic or alicyclic (i.e., carbocycle);
- heteroaryl is a hydrocarbon group containing at least one heteroatom and at least one aromatic ring; The number of carbons and the number and type of heteroatom are indicated at each occurrence of the term. Heteroaryl may also contain more than one aromatic ring, then these rings may be condensed or non-fused;
- aryloxy refers to a group -OR', where R' is a C6-C16 aryl as defined herein above.

As used herein, the term "pharmaceutically acceptable salts" refers to salts that retain the biological effectiveness and properties of the claimed compounds of general formula I according to this invention, and which are within reasonable medical judgment suitable for use in contact with the tissues of humans and lower animals without undue toxicity, irritation, allergic reactions, and the like, and have an acceptable benefit/risk ratio. In many cases, the compounds of the present invention are capable of forming acid and/or base salts by virtue of the presence of amino and/or phosphoric acid groups. Pharmaceutically acceptable acid addition salts can be formed with inorganic acids and organic acids. Inorganic acids from which salts can be derived include, for example, hydrochloric acid, hydrobromic acid, sulfuric acid, nitric acid, phosphoric acid, and the like. Organic acids from which salts can be derived include, for example, acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, *p*-toluenesulfonic acid, salicylic acid, and the like. Pharmaceutically acceptable base addition salts can be formed with inorganic and organic bases. Inorganic bases from which salts can be derived include, for example, sodium, potassium, lithium, ammonium, calcium, magnesium, iron, zinc, copper, manganese, aluminium, and the like; particularly preferred are the ammonium, potassium, sodium, calcium and magnesium salts. Organic bases from which salts can be derived include, for example, primary, secondary, and tertiary amines, substituted amines including naturally occurring substituted amines, cyclic amines, basic ion exchange resins, and the like, specifically such as isopropylamine, trimethylamine, diethylamine, triethylamine, tripropylamine, and ethanolamine. The pharmaceutically acceptable salts of the present invention can be synthesized from a parent compound, a basic or acidic moiety, by conventional chemical methods. Generally, such salts can be prepared by reacting free acid forms of these compounds with a stoichiometric amount of the appropriate base (such as Na, Ca, Mg, or K hydroxide, carbonate, bicarbonate, or the like), or by reacting free base forms of these compounds with a stoichiometric amount of the appropriate acid. Such reactions are typically carried out in water or in an organic solvent, or in a mixture of the two. Generally, non-aqueous media like ether, ethyl acetate, ethanol, isopropanol, or acetonitrile are preferred, where practicable. Lists of additional suitable salts can be found, e.g., in Remington's Pharmaceutical Sciences, 20th ed., Mack Publishing Company, Easton, Pa., (1985), which is herein incorporated by reference.

In a preferred embodiment, the present invention provides 7-substituted 7-deazaadenine-containing CDNs of general formula I, being:
2-amino-9-((5*R*,7*R*,8*R*,12a*R*,14*R*,15*R*,15a*S*,16*R*)-14-(4-amino-5-methyl-7*H*-pyrrolo[2,3-*d*]pyrimidin-7-yl)-2,10,15,16-tetrahydroxy-2,10-dioxidooctahydro-12*H*-5,8-methanofuro[3,2-*l*] [1,3,6,9,11]pentaoxa[2,10]diphosphacyclotetradecin-7-yl)-1,9-dihydro-6*H*-purin-6-one,
2-amino-9-((5*R*,7*R*,8*R*,12a*R*,14*R*,15*R*,15a*S*,16*R*)-14-(4-amino-5-phenyl-7*H*-pyrrolo[2,3-*d*]pyrimidin-7-yl)-2,10,15,16-tetrahydroxy-2,10-dioxidooctahydro-12*H*-5,8-methanofuro[3,2-*l*] [1,3,6,9,11]pentaoxa[2,10]diphosphacyclotetradecin-7-yl)-1,9-dihydro-6*H*-purin-6-one,
2-amino-9-((5*R*,7*R*,8*R*,12a*R*,14*R*,15*R*,15a*S*,16*R*)-14-(4-amino-5-(naphthalen-2-yl)-7*H*-pyrrolo[2,3-*d*]pyrimidin-7-yl)-2,10,15,16-tetrahydroxy-2,10-dioxidooctahydro-12*H*-5,8-methanofuro[3,2-*l*] [1,3,6,9,11]pentaoxa[2,10]diphosphacyclotetradecin-7-yl)-1,9-dihydro-6*H*-purin-6-one,
2-amino-9-((5*R*,7*R*,8*R*,12a*R*,14*R*,15*R*,15a*S*,16*R*)-14-(4-amino-5-(naphthalen-1-yl)-7*H*-pyrrolo[2,3-*d*]pyrimidin-7-yl)-2,10,15,16-tetrahydroxy-2,10-dioxidooctahydro-12*H*-5,8-methanofuro[3,2-*l*] [1,3,6,9,11]pentaoxa[2,10]diphosphacyclotetradecin-7-yl)-1,9-dihydro-6*H*-purin-6-one,
2-amino-9-((5*R*,7*R*,8*R*, 12a*R*, 14*R*, 15*R,* 15a*S*, 16*R*)- 14-(4-amino-5 -(phenanthren-9-yl)-7*H*-pyrrolo[2,3 - *d*]pyrimidin-7-yl)-2,10,15,16-tetrahydroxy-2,10-dioxidooctahydro-12*H*-5,8-methanofuro[3,2-*l*] [1,3,6,9,11]pentaoxa[2,10]diphosphacyclotetradecin-7-yl)-1,9-dihydro-6*H*-purin-6-one,
9*-*((5*R*,7*R,*8*R,*12a*R,*14*R,*15*R,*15a*S,*16*R*)*-*14-(5-([1*,*1*'*-biphenyl]-4-yl)-4-arnino-7*H*-pyrrolo[2,3-*d*]pyrimidin-7-yl)-2,10,15,16-tetrahydroxy-2,10-dioxidooctahydro-12*H*-5,8-methanofuro[3,2-*l*] [1,3,6,9,11]pentaoxa[2,10]diphosphacyclotetradecin-7-yl)-2-amino-1,9-dihydro-6*H*-purin-6-one,
2-amino-9-((5*R*,7*R*,8*R*,12a*R*,14*R*, 15*R,* 15a*S*, 16*R*)-14-(4-amino-5-(4-(naphthalen-2-yl)phenyl)-7*H-*pyrrolo[2,3-*d*]pyrimidin-7-yl)-2,10,15,16-tetrahydroxy-2,10-dioxidooctahydro-12*H*-5,8-methanofuro[3,2-*l*] [1,3,6,9,11]pentaoxa[2,10]diphosphacyclotetradecin-7-yl)-1,9-dihydro-6*H*-purin-6-one,
2-amino-9-((5*R*,7*R*,8*R*, 12a*R*, 14*R*, 15*R,* 15a*S*,16*R*)-14-(4-amino-5-(4-((naphthalen-1-yloxy)methyl)phenyl)-7*H*-pyrrolo[2,3-*d*]pyrimidin-7-yl)-2,10,15,16-tetrahydroxy-2,10-dioxidooctahydro-12*H*-5,8-methanofuro[3,2-*l*] [1,3,6,9,11]pentaoxa[2,10]diphosphacyclotetradecin-7-yl)-1,9-dihydro-6*H*-purin-6-one,
2-amino-9-((5*R*,7*R*,8*R*,12a*R*, 14*R*, 15*R,* 15a*S*, 16*R*)- 14-(4-amino-5-(furan-2-yl)-7*H*-pyrrolo[2,3-*d*]pyrimidin-7-yl)-2,10,15,16-tetrahydroxy-2,10-dioxidooctahydro-12*H*-5,8-methanofuro[3,2-*l*] [1,3,6,9,11]pentaoxa[2,10]diphosphacyclotetradecin-7-yl)-1,9-dihydro-6*H*-purin-6-one,
2-amino-9-((5*R*,7*R*,8*R*, 12a*R*, 14*R*, 15*R,* 15a*S*, 16*R*)-14-(4-amino-5-(thiophen-2-yl)-7*H*-pyrrolo[2,3 - *d*]pyrimidin-7-yl)-2,10,15,16-tetrahydroxy-2,10-dioxidooctahydro-12*H*-5,8-methanofuro[3,2-*l*] [1,3,6,9,11]pentaoxa[2,10]diphosphacyclotetradecin-7-yl)-1,9-dihydro-6*H*-purin-6-one,
2-amino-9-((5*R*,7*R*,8*R*,12a*R*,14*R*,15*R*,15a*S*,16*R*)-14-(4-amino-5-(benzo[*b*]furan-2-yl)-7*H*-pyrrolo[2,3 - *d*]pyrimidin-7-yl)-2,10,15,16-tetrahydroxy-2,10-dioxidooctahydro-12*H*-5,8-methanofuro[3,2-*l*] [1,3,6,9,11]pentaoxa[2,10]diphosphacyclotetradecin-7-yl)-1,9-dihydro-6*H*-purin-6-one,
2-amino-9-((5*R*,7*R*,8*R*,12a*R*,14*R*,15*R*,15a*S*,16*R*)-14-(4-amino-5-(benzo[*b*]thiophen-2-yl)-7*H*-pyrrolo[2,3-*d*]pyrimidin-7-yl)-2,10,15,16-tetrahydroxy-2,10-dioxidooctahydro-12*H*-5,8-methanofuro[3,2-*l*] [1,3,6,9,11]pentaoxa[2,10]diphosphacyclotetradecin-7-yl)-1,9-dihydro-6*H*-purin-6-one,
2-amino-9-((5*R*,7*R*,8*R*,12a*R*,14*R*,15*R*,15a*S*,16*R*)-14-(4-amino-5-(dibenzo[*b,d*]furan-4-yl)-7*H*-pyrrolo [2,3-*d*]pyrimidin-7-yl)-2,10,15,16-tetrahydroxy-2,10-dioxidooctahydro-12*H*-5,8-methanofuro[3,2-*l*] [1,3,6,9,11]pentaoxa[2,10]diphosphacyclotetradecin-7-yl)-1,9-dihydro-6*H*-purin-6-one,
and pharmaceutically acceptable salts thereof.

Additionally, the present invention provides the 7-substituted 7-deazaadenine-containing CDNs of general formula **I** or pharmaceutically acceptable salts thereof for use as medicaments.

Furthermore, the present invention provides the 7-substituted 7-deazaadenine-containing CDNs of general formula **I** or pharmaceutically acceptable salts thereof for use in the treatment or prevention of a viral infection, such as hepatitis B virus infection or HIV infection, a hyperproliferative disease or cancer.

Yet furthermore, the present invention provides the 7-substituted 7-deazaadenine-containing CDNs of general formula I or pharmaceutically acceptable salts thereof for use as an adjuvans to a vaccine to enhance the efficacy of the vaccine.

The present disclosure includes a pharmaceutical composition comprising the cyclic dinucleotide of formula I, or an enantiomer or a mixture of enantiomers, or a pharmaceutically acceptable salt thereof, and one or more pharmaceutically acceptable carriers, excipients, and/or diluents.

Also provided is a method of treating or preventing a disease or disorder, e.g., a method of treating or preventing a viral infection, hepatitis B virus infection, HIV infection, hyperproliferative disease or cancer, comprising administering to a human or animal in need thereof a therapeutically effective amount of a cyclic dinucleotide of formula I, or an enantiomer or a mixture of enantiomers, or a pharmaceutically acceptable salt thereof, or a pharmaceutical composition of any of the foregoing.

Further provided is a method of enhancing the efficacy of a vaccine, comprising administering a therapeutically effective amount of a cyclic dinucleotide of formula I, or an enantiomer or a mixture of enantiomers, or a pharmaceutically acceptable salt thereof, or a pharmaceutical composition of any of the foregoing.

Additionally, provided herein is a method of modulating the activity of STING adaptor protein to induce production of type I interferon, cytokine and/or chemokine dependent on the STING adaptor protein, e.g., inducing a STING adaptor protein-dependent type I interferon, cytokine or chemokine in a human or animal, comprising administering a therapeutically effective amount of a cyclic dinucleotide of formula **I,** or an enantiomer, or pharmaceutically acceptable salt thereof, or a pharmaceutical composition of any of the foregoing.

"Therapeutically effective amount" or "effective amount" as used herein refers to an amount that is effective to elicit the desired biological or medical response, including the amount of a compound that, when administered to a subject for treating a disease, is sufficient to effect such treatment for the disease. The effective amount will vary depending on the compound, the disease, and its severity and the age, weight, etc., of the subject to be treated. The effective amount can include a range of amounts. As is understood in the art, an effective amount may be in one or more doses, i.e., a single dose or multiple doses may be required to achieve the desired treatment endpoint. An effective amount may be considered in the context of administering one or more therapeutic agents, and a single agent may be considered to be given in an effective amount if, in conjunction with one or more other agents, a desirable or beneficial result may be or is achieved. Suitable doses of any co-administered compounds may optionally be lowered due to the combined action (e.g., additive or synergistic effects) of the compounds.

In some embodiments, a therapeutically effective amount of a compound provided herein or pharmaceutically acceptable salt thereof may (i) reduce the number of diseased cells; (ii) reduce tumor size; (iii) inhibit, retard, slow to some extent, and preferably stop the diseased cell infiltration into peripheral organs; (iv) inhibit (e.g., slow to some extent and preferably stop) tumor metastasis; (v) inhibit tumor growth; (vi) prevent or delay occurrence and/or recurrence of a tumor, and/or (vii) relieve to some extent one or more of the symptoms associated with cancer or hyperproliferative disease. In some embodiments, a therapeutically effective amount is sufficient to ameliorate, palliate, lessen, and/or delay one or more symptoms of cancer or hyperproliferative disease.

The term "pharmaceutical composition" refers to the formulation of a compound and medium, generally accepted in the art, for the delivery of a biologically active compound to a mammal, e.g., a human. Such a medium includes all pharmaceutically acceptable carriers, diluents or excipients.

The term "pharmaceutically acceptable carrier, diluent or filler" as used herein includes, without limitation, any excipient, carrier, glidant, sweetener, preservative, dye, flavour enhancer, surfactant, dispersing agent, suspending agent, isotonic agent, solvent, or emulsifier that has been approved for use in humans or domestic animals.

The invention further relates to compounds of formula I for use as an active ingredient in a pharmacologically acceptable composition which may be prepared by conventional methods known in the art, e.g., the active ingredient may be in admixture with pharmaceutically acceptable inert organic and/or inorganic carriers and/or with auxiliaries or, where appropriate, attached to them.

In one embodiment, the present invention also relates to the use of compounds of formula I as prodrugs or other suitable forms which release the active ingredient *in vivo.*

### Examples

**Table 1: List of Compounds in Examples**

| Example (compound) | Structure | Systematic name |
|---|---|---|
| 5 (**1a**) | 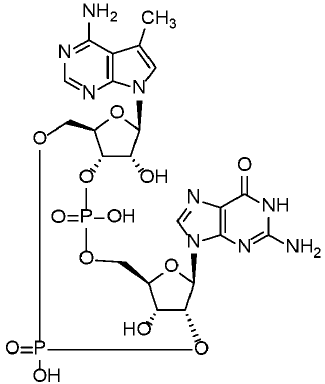 | 2-amino-9-((5*R*,7*R*,8*R,*12a*R*,14*R,*15*R,*15a*S,*16*R*)*-*14-(4-amino-5-methyl-7*H*-pyrrolo[2,3-*d*]pyrimidin-7-yl)-2,10,15,16-tetrahydroxy-2,10-dioxidooctahydro-12*H-*5,8-methanofuro[3,2-*l*] [1,3,6,9, 11]pentaoxa[2,10] diphosphacyclotetradecin -7-yl)-1,9-dihydro-6*H*-purin-6-one |
| 6 (**1b**) | 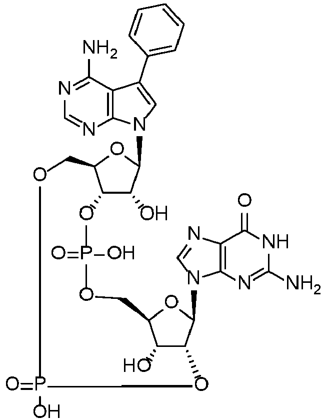 | 2-amino-9-((5*R*,7*R*,8*R*,12a*R*,14*R*,15*R*,15a*S*,16*R*)-14-(4-amino-5-phenyl-7*H*-pyrrolo[2,3-*d*]pyrimidin-7-yl)-2,10,15,16-tetrahydroxy-2,10-dioxidooctahydro-12*H-*5,8-methanofuro[3,2-*l*][1,3,6,9,11]pentaoxa[2,10]diphosphacyclotetradecin -7-yl)-1,9-dihydro-6*H*-purin-6-one |
| 7 (**1c**) | 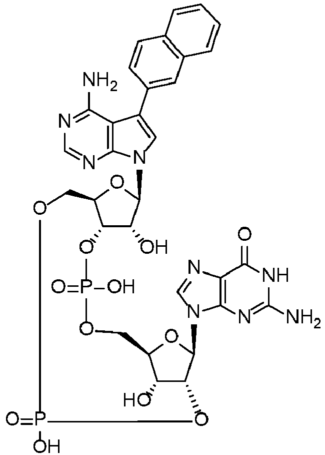 | 2-amino-9-((5*R*,7*R*,8*R*,12a*R*,14*R*,15*R*,15a*S*,16*R*)-14-(4-amino-5-(naphthalen-2-yl)-7*H*-pyrrolo[2,3-*d*]pyrimidin-7-yl)-2,10,15,16-tetrahydroxy-2,10-dioxidooctahydro-12*H*-5,8-methanofuro[3,2-*l*][1,3,6,9,11]pentaoxa[2,10]diphosphacyclotetradecin -7-yl)-1,9-dihydro-6*H*-purin-6-one |
| 8 (**1d**) | 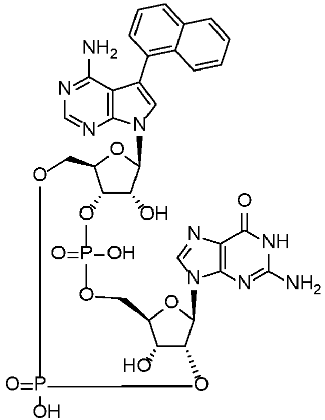 | 2-amino-9-((5*R,*7*R,*8*R,*12a*R,*14*R,*15*R,*15a*S,*16*R)-*14-(4-amino-5-(naphthalen-1-yl)-7*H*-pyrrolo[2,3-*d*]pyrimidin-7-yl)-2,10,15,16-tetrahydroxy-2,10-dioxidooctahydro-12*H*-5,8-methanofuro[3,2-*l*][1,3,6,9,11]pentaoxa[2,10] diphosphacyclotetradecin -7-yl)-1,9-dihydro-6*H*-purin-6-one |
| 9 (**1e**) | 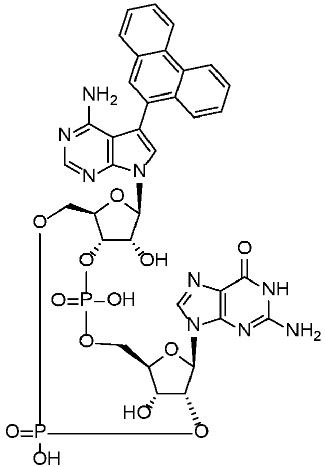 | 2-amino-9-((5*R*,7*R*,8*R*,12a*R*,14*R*,15*R*,15a*S,*16*R*)-14-(4-amino-5-(phenanthren-9-yl)-7*H*-pyrrolo[2,3-*d*]pyrimidin-7-yl)-2,10,15,16-tetrahydroxy-2,10-dioxidooctahydro-12*H*-5,8-methanofuro[3,2-*l*][1,3,6,9,11]pentaoxa[2,10] diphosphacyclotetradecin -7-yl)-1,9-dihydro-6*H*-purin-6-one |
| 10 (**1f**) | 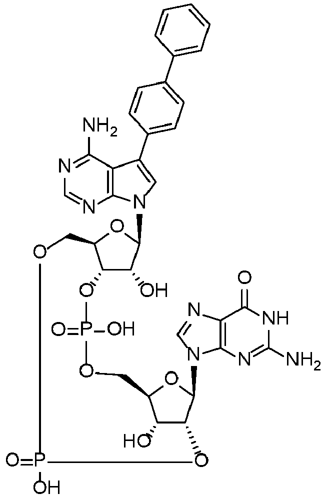 | 9-((5*R*,7*R*,8*R*,12a*R*,14*R*,15*R*,15a*S*,16*R*)-14-(5-([1,1'-biphenyl]-4-yl)-4-amino-7*H*-pyrrolo[2,3-*d*]pyrimidin-7-yl)-2,10,15,16-tetrahydroxy-2,10-dioxidooctahydro-12*H*-5,8-methanofuro[3,2-*l*][1,3,6,9,11]pentaoxa[2,10]diphosphacyclotetradecin -7-yl)-2-amino-1,9-dihydro-6*H*-purin-6-one |
| 11 (**1g**) | 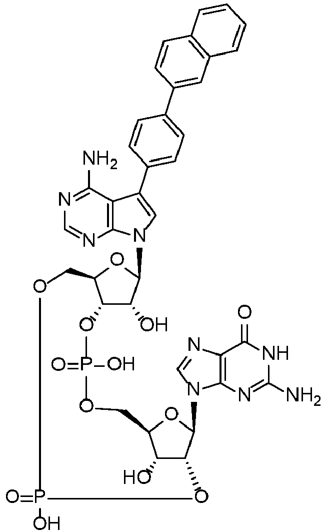 | 2-amino-9-((5*R*,7*R*,8*R*,12a*R*,14*R*,15*R*,15a*S*,16*R*)-14-(4-amino-5-(4-(naphthalen-2-yl)phenyl)-7*H-*pyrrolo[2,3-*d*]pyrimidin-7-yl)-2,10,15,16-tetrahydroxy-2,10-dioxidooctahydro-12*H*-5,8-methanofuro[3,2-*l*][1,3,6,9,11]pentaoxa[2,10] diphosphacyclotetradecin -7-yl)-1,9-dihydro-6*H*-purin-6-one |
| 12 (**1h**) | 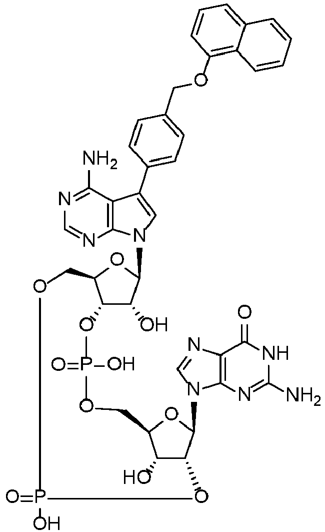 | 2-amino-9-((5*R*,7*R*,8*R*,12a*R*,14*R,*15*R,*15a*S,*16*R*)-14-(4-amino-5-(4-((naphthalen-1-yloxy)methyl)phenyl)-7*H*-pyrrolo[2,3-*d*]pyrimidin-7-yl)-2,10,15,16-tetrahydroxy-2,10-dioxidooctahydro-12*H*-5,8-methanofuro[3,2-*l*][1,3,6,9,11]pentaoxa[2,10]diphosphacyclotetradecin -7-yl)-1,9-dihydro-6*H*-purin-6-one |
| 13 (**1i**) | 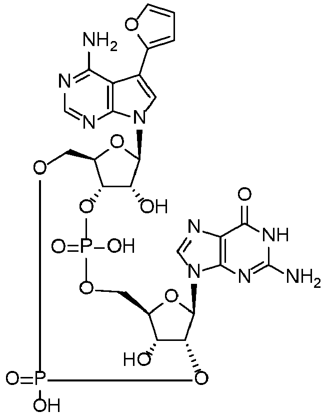 | 2-amino-9-((5*R*,7*R*,8*R*,12a*R*,14*R*,15*R*,15a*S*,16*R*)-14-(4-amino-5-(furan-2-yl)-7*H*-pyrrolo[2,3-*d*]pyrimidin-7-yl)-2,10,15,16-tetrahydroxy-2,10-dioxidooctahydro-12*H*-5,8-methanofuro[3,2-*l*][1,3,6,9,11]pentaoxa[2,10] diphosphacyclotetradecin -7-yl)-1,9-dihydro-6*H*-purin-6-one |
| 14 (**1j**) | 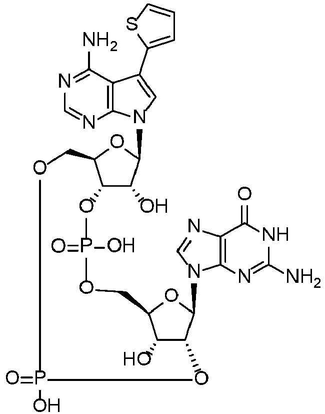 | 2-amino-9-((5*R*,7*R*,8*R*,12a*R*,14*R*,15*R*,15a*S*,16*R*)-14-(4-amino-5-(thiophen-2-yl)-7*H*-pyrrolo[2,3-*d*]pyrimidin-7-yl)-2,10,15,16-tetrahydroxy-2,10-dioxidooctahydro-12*H*-5,8-methanofuro[3,2-*l*][1,3,6,9,11]pentaoxa[2,10] diphosphacyclotetradecin -7-yl)-1,9-dihydro-6*H*-purin-6-one |
| 15 (**1k**) | 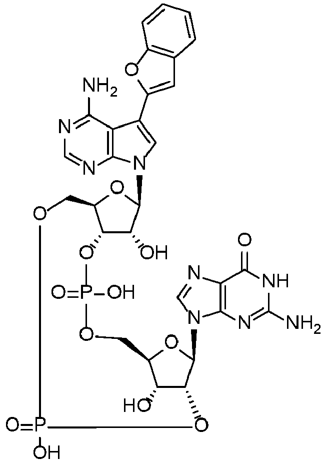 | 2-amino-9-((5*R*,7*R*,8*R*,12a*R*,14*R*,15*R*,15a*S*,16*R*)-14-(4-amino-5 -(benzo[*b*]furan-2-yl)-7*H*-pyrrolo [2,3-*d*]pyrimidin-7-yl)-2,10,15,16-tetrahydroxy-2,10-dioxidooctahydro-12*H*-5,8-methanofuro[3,2-*l*][1,3,6,9,11]pentaoxa[2,10]diphosphacyclotetradecin -7-yl)-1,9-dihydro-6*H*-purin-6-one |
| 16 (**1l**) | 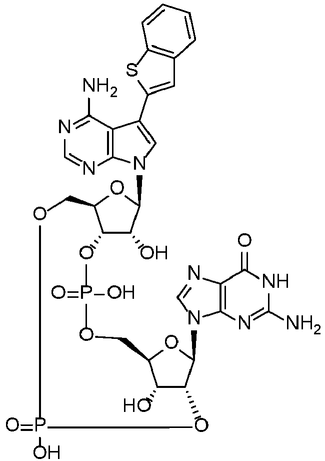 | 2-amino-9-((5*R*,7*R*,8*R*,12a*R*,14*R*,15*R*,15a*S*,16*R*)-14-(4-amino-5-(benzo[*b*]thiophen-2-yl)-7*H*-pyrrolo[2,3-*d*]pyrimidin-7-yl)-2,10,15,16-tetrahydroxy-2,10-dioxidooctahydro-12*H*-5,8-methanofuro[3,2-*l*][1,3,6,9,11]pentaoxa[2,10] diphosphacyclotetradecin -7-yl)-1,9-dihydro-6*H*-purin-6-one |
| 17 (**1m**) | 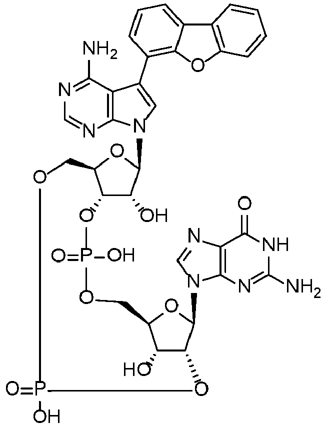 | 2-amino-9-((5*R*,7*R*,8*R*,12a*R*,14*R*,15*R*,15a*S*,16*R*)-14-(4-amino-5-(dibenzo[*b,d*]furan-4-yl)-7*H*-pyrrolo[2,3-*d*]pyrimidin-7-yl)-2,10,15,16-tetrahydroxy-2,10-dioxidooctahydro-12*H*-5,8-methanofuro[3,2-*l*][1,3,6,9,11]pentaoxa[2,10]diphosphacyclotetradecin -7-yl)-1,9-dihydro-6*H*-purin-6-one |
| comparative compound (**1n**) | 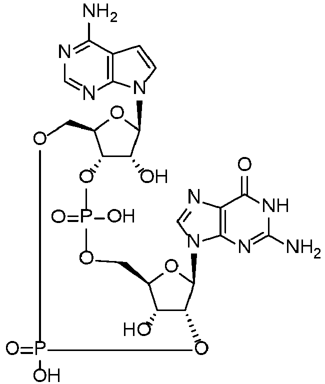 | 2-amino-9-((5*R*,7*R*,8*R*,12a*R*,14*R*,15*R*,15a*S*,16*R*)-14-(4-amino-7*H*-pyrrolo[2,3-*d*]pyrimidin-7-yl)-2,10,15,16-tetrahydroxy-2,10-dioxidooctahydro-12*H-*5,8-methanofuro[3,2-*l*][1,3,6,9,11]pentaoxa[2,10] diphosphacyclotetradecin -7-yl)-1,9-dihydro-6*H*-urin-6-one |

### Synthesis of compounds

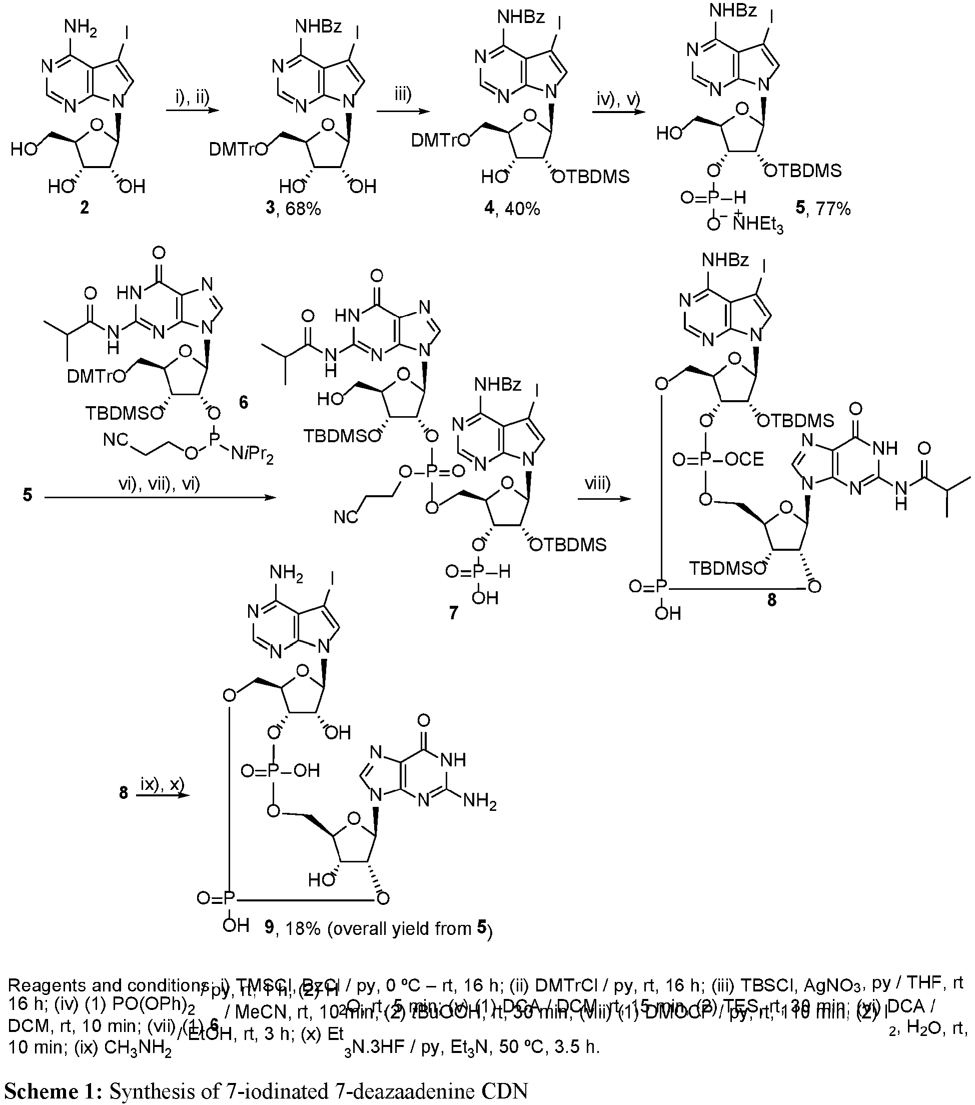

The key intermediate, 7-iodo-7-deazaadenine CDN 9 can be prepared by chemical synthesis or by enzymatic synthesis. The chemical synthesis of iodinated 7-deazaadenine CDN **9** started from 7-iodo-7-deazaadenosine **(2)** (Scheme 1). Iodinated nucleoside **2** was *N*-benzoylated using transient silyl protection (Zhu, X.-F. et al. Synth. Commun. 2003, 33, 1233-1243). The crude product was directly used in a tritylation step to obtain 5'-O-DMTr-protected nucleoside **3.** Regioselective 2'-*O*-silylation using silver nitrate catalysis (Hakimelahi, G. H. et al. Can. J. Chem. 1982, 60, 1106-1113) provided nucleoside **4.** 3'*-H-*Phosphonate moiety was installed by reaction of **4** with diphenyl phosphite. The crude product was directly used for the next step in order to avoid loss of the material during column chromatography. After removal of the DMTr-group by dichloroacetic acid, *H*-phosphonate **5** was obtained as a triethylammonium salt. After pre-treatment of H-phosphonate **5** with dichloroacetic acid, it was coupled with guanosine phosphoramidite **6** in acetonitrile. After detritylation, linear dinucleotide **7** was partially purified using reverse phase flash chromatography. The crude linear product **7** was cyclized using DMOCP (Gaffney, B. L. et al Org. Lett. 2010, 12, 3269-3271) and after oxidation with iodine and partial purification on reverse phase C18 column, the protected cyclic dinucleotide **8** was obtained as a crude material. Deprotection of nucleobases using methylamine and removal of silyl groups by Et₃N. 3HF provided 7-iodinated 7-deazaadenine CDN **9.**

Enzymatical synthesis of CDNs depended on mcGAS. 7-Substituted 7-deazaadenosine triphosphates **10-16** (Bourderioux, A. et al. J. Med. Chem. 2011, 54, 5498-5507; Milisavljevič, N. et al. Org. Biomol. Chem. 2018, 16, 5800-5807; Milisavljevic, N. et al. ACS Infect. Dis. 2021, 7, 471-478) were used as substrates for enzymatic cyclization with GTP using mcGAS to provide CDNs **1a,b,i,j** and 9 (Scheme 2, Table 2).

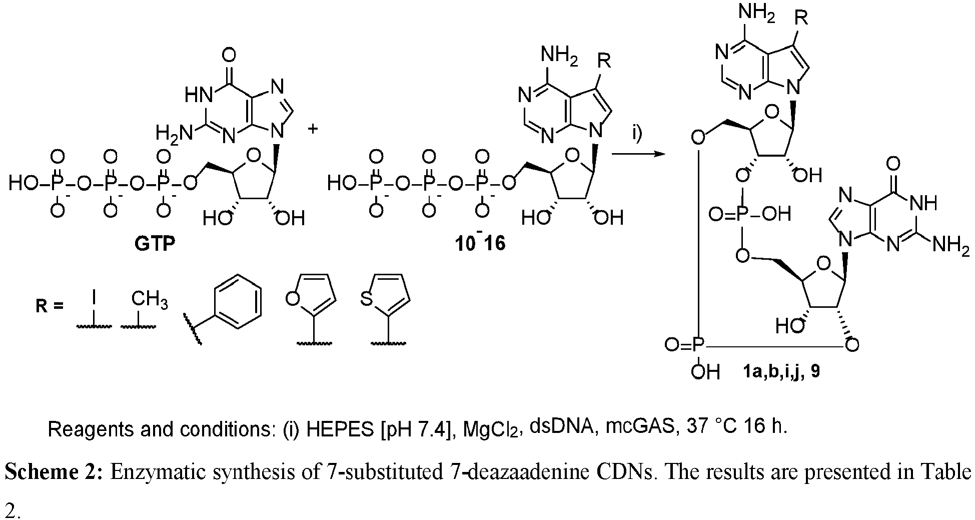

**Table 2: Enzymatic synthesis of 7-substituted 7-deazaadenine CDNs**

| Entry | Triphosphate | R | Product |
|---|---|---|---|
| 1 | **10** | methyl | **1a** |
| 2 | **11** | phenyl | **1b** |
| 3 | **12** | furan-2-yl | **1i** |
| 4 | **13** | thiophen-2-yl | **1j** |
| 5 | **14** | iodo | **9** |

7-Aryl- and 7-hetaryl-7-deazaadenine CDNs **1b-h,k-m** were synthesized using Suzuki-Miyaura cross-coupling reaction from 7-iodinated CDN **9.** The cross-coupling reactions were performed under Pd(OAc)₂ catalysis in the presence of a water-soluble ligand, triphenylphosphan-3,3',3"-trisulfonate (TPPTS), and CS₂CO₃ in water-acetonitrile mixture (2:1) (Scheme 4). Due to hindered rotation at room temperature, CDNs **1d** and **1e** were prepared as inseparable mixtures of diastereomers/atropoisomers.

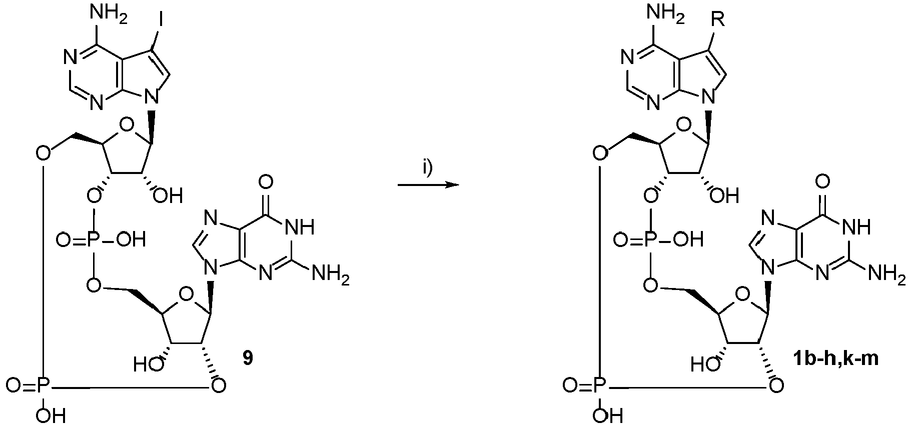

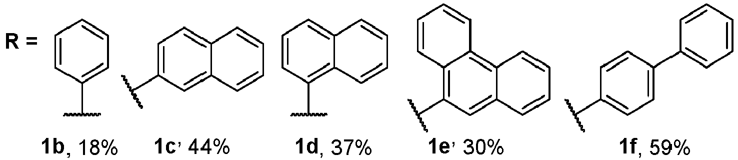

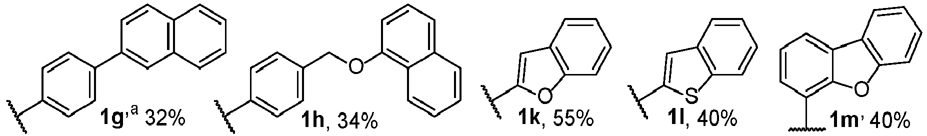

Reagents and conditions: (i) RB(OH)_{2'} Cs₂CO₃, TPPTS, Pd(OAc)₂ /H ₂O-MeCN (2:1), 100 °C, 30 min. ^{a}RBpin was used instead of RB(OH)₂

### List of abbreviations

- Ac: acetyl
- AMP: adenosine 5'-monophosphate
- ATP: adenosine 5'-triphosphate
- bd: broad doublet
- bdd: broad doublet of doublets
- bs: broad singlet
- bdt: broad doublet of triplets
- Bu: butyl
- Bz: benzoyl
- calcd: calculated
- CDN: cyclic dinucleotide
- cGAMP: cyclic guanosine monophosphate-adenosine monophosphate
- d: doublet
- DCA: dichloroacetic acid
- DCM: dichloromethane
- dd: doublet of doublets
- ddd: doublet of doublet of doublets
- ddH₂O: double-distilled water
- dm: doublet of multiplets
- DMEM: Dulbecco's Modified Eagle Medium
- DMF: *N,N*-dimethylformamide
- DMOCP: 2-chloro-5,5-dimethyl-1,3,2-dioxaphosphorinane 2-oxide
- DMTr: 4,4'-dimethyltrityl
- DNA: deoxyribonucleic acid
- dsDNA: double-stranded deoxyribonucleic acid
- dt: doublet of triplets
- DTT: 1,4-dithiothreitol
- EDTA: ethylenediaminetetraacetic acid
- eq.: equivalent
- ESI: electrospray ionization
- Et: ethyl
- EtOAc: ethyl acetate
- EtOH: ethanol
- Et₃N: trimethylamine
- FBS: fetal bovine serum
- GTP: guanosine 5'-triphosphate
- HEPES: 4-(2-hydroxyethyl)piperazine-l-ethanesulfonic acid
- HPFC: high-performance flash chromatography
- HPLC: high-performance liquid chromatography
- HR: high resolution
- hSTING: human stimulator of interferon genes
- IFN: interferon
- IPTG: isopropyl β-_{D}-1-thiogalactopyranoside
- iPr: isopropyl
- m: multiplet
- mcGAS: mouse cyclic GMP-AMP synthase
- Me: methyl
- MeCN: acetonitrile
- MeOH: methanol
- MS: mass spectrometry
- naphth: naphthyl
- Ni-NTA: nickel-nitrilotriacetic acid
- NMR: nuclear magnetic resonance
- OCE: 2-cyanoethyloxy
- PBS: phosphate buffered saline
- pin: pinacolato
- Ph: phenyl
- phen: phenanthrenyl
- PMBC: peripheral blood mononuclear cells
- py: pyridine
- q: quartet
- qt: quartet of triplets
- rt: room temperature
- s: singlet
- STING: stimulator of interferon genes
- t: triplet
- TBDMS: *tert*-butyldimethylsilyl
- TBSC1: *tert*-butyldimethylsilyl chloride
- *t*BuOOH: *tert-*butyl hydroperoxide
- td: triplet of doublets
- TEAB: triethylammonium bicarbonate
- TES: triethylsilane
- THF: tetrahydrofuran
- TMSC1: trimethylsilyl chloride
- TPPTS: triphenylphosphan-3,3',3"-trisulfonate
- Tris: tris(hydroxymethyl)aminomethane
- v/v: volume/volume
- um: unresolved multiplet
- *wt*: wild type
- wt.: weight

NMR spectra were measured on 500 MHz spectrometer (499.8 MHz for ¹H. 125.7 MHz for ¹³C and ³¹P at 202.4 MHz) in D₂O (dioxane used as external standard, [δ(¹H) = 3.75 ppm, δ(¹³C) = 67.19 ppm]) or in CD₃OD (referenced to the residual solvent signal, [δ(¹H) = 3.31 ppm, δ(¹³C) = 49.0 ppm]). ³¹P NMR spectra were referenced externally to the signal of H₃PO₄. Chemical shifts are given in ppm (δ-scale), coupling constants (J) are given in Hz. Low resolution mass spectra were measured using electrospray ionization (ESI). High resolution mass spectra were measured using ESI. High performance flash chromatography (HPFC) were performed with ISCO Combiflash Rf system on RediSep Rf Gold Silica Gel columns or Reverse Phase (C18) RediSep Rf Gold columns. Purification of CDNs was performed using HPLC (Waters modular HPLC system) on a column packed with 5 µm polar C18 reversed phase (Luna Omega 5 µm Polar C18 column, Phenomenex). Purity of all final compounds (>95%) was determined by clean NMR spectra and by UPLC.

### Production and purification of mcGAS

The expression and purification of mcGAS was performed as previously described (Novotná, B. et al. J. Med. Chem. 2019, 62, 10676-10690) with minor changes. The plasmid (pET22b) encoding truncated mouse cGAS (AA 147-507) was transformed in *E*. *coli* BL21 (DE3) cells (ThermoFisher, USA). The bacteria were grown at 37 °C until OD₆₀₀ₙₘ of 0.6-0.8 units, then cooled to 20 °C, induced with 0.4 mM IPTG and incubated for 16 h in shaker. After cell collection, pellet was re-suspended in ice-cold lysis/wash buffer (50 mM Tris-HCl [pH 8], 300 mM NaCl, 20 mM imidazole, 10% (w/v) glycerol, 3 mM β-mercaptoethanol), cells were lysed by sonication and cell debris were removed by centrifugation. The lysate supernatants were incubated with equilibrated Ni-NTA beads (Macherey-Nagel, Germany). Bead were loaded into a gravity flow column, washed with lysis/wash buffer, then with lysis/wash buffer supplemented with NaCl to 1 M concentration. After extra wash with lysis/wash buffer, mouse cGAS was eluted with lysis/wash buffer supplemented with imidazole to 300 mM concentration. Eluted mcGAS was further purified on sizeexclusion chromatography column HiLoad 26/60 Superdex 200 equilibrated with buffer 50 mM Tris-HCl [pH 7.4] and 150 mM NaCl. Purified protein was concentrated to about 5 mg/mL, and stored in 20 % glycerol at -80 °C.

### General procedure A (Enzymatic synthesis of CDNs)

A nucleoside triphosphate **10-14** and GTP were mixed to final 2 mM concentration with 5 µM mcGAS and 0.1 mg/mL herring testes DNA in 1 mL of buffer containing 20 mM Tris-HCl [pH 8.0] and 20 mM MgCl₂. After 16 h incubation at 37 °C in heating shaker, the reactions were spun at 25000×g for 15 min, and supernatants were passed through Nanosep 3K Omega (Pall Corporation, USA). Prepared CDNs present in flow-through were diluted with 3 mL of ddH₂O and purified on semipreparative C 18 column (Luna 5 µm C18, 250 mm × 10 mm) using 60 min gradient at flow rate 3 mL/min of 0-20% acetonitrile in 0.1 M TEAB buffer [pH 8.5]. TEAB was removed from the collected fractions by three cycles of evaporation/dissolution in 50% methanol.

### General procedure B (Suzuki-Miyaura cross-coupling):

A cyclic dinucleotide 9 (13 mg, 15.4 µmol), boronic acid (77.1 µmol) and cesium carbonate (15 mg, 46.2 µmol) were mixed with MeCN-H₂O (1:2v/v, 520 µl) in an argon purged vial. In a separate vial Pd(OAc)₂ (1.0 mg, 4.45 µmol) and TPPTS (12.6 mg, 22.2 µmol) were dissolved in MeCN-H₂O (1:2v/v, 1.0 ml) and the solution was sonicated under argon atmosphere for 30s. Then 173 µmol of this solution was transferred into the mixture containing the cyclic dinucleotide 9 and the reaction was stirred at 100 °C for 30 min. Then the reaction mixture was cooled to rt, diluted with water to approx. 3 ml and filtered through a 5 µm nylon syringe filter. The filtrate was directly applied on HPLC for purification.

### Example 1

### 4-Benzamido-5-iodo-7-[5-O-(4,4'-dimethoxytrityl)-β-D-ribofuranosyl]-7H-pyrrolo[2,3-d]pyrimidine (3).

7-Iodo-7-deazaadenosine (2, 500 mg, 1.28 mmol) was co-evaporated with anhydrous pyridine (2× 3 ml) and suspended in anhydrous pyridine (5 ml). The suspension was cooled to 0 °C and trimethylsilyl chloride (728 µl, 5.74 mmol) was added dropwise over a period of 30 min. Then, benzoyl chloride (222 µl, 1.91 mmol) was added and the mixture was stirred at rt overnight. The reaction mixture was then cooled to 0 °C and water (1.25 ml) was added. After stirring for 15 min, aqueous ammonia (25%, 2.5 ml) was added and the stirring was continued for another 30 min. Solvents were then evaporated and the residue was co-evaporated with anhydrous pyridine (2× 3 ml) and dissolved in anhydrous pyridine (5 ml). DMTrCl (432 mg, 1.25 mmol) was added in two portions over 1 h and the reaction mixture was stirred overnight. Then DMTrCl (432 mg, 1.25 mmol) was added again in two portions over 1 h and the reaction mixture was stirred for another 1.5 h. The solvent was removed in vacuo, the residue was suspended in DCM (35 ml) and solid phase was filtered off through a pad of celite. The filtrate was washed with saturated aqueous NaHCO₃, dried over magnesium sulfate and evaporated. Flash chromatography on silica (0-10 % MeOH in DCM + 1% Et₃N) provided nucleoside 3 (689 mg, 68 %) as a white amorphous solid. ¹H NMR (500.0 MHz, CD₃OD): 3.39 (dd, 1H, *J*_{gem} = 10.7, *J*_{5'b,4'} = 2.9, H-5'b); 3.42 (dd, 1H, *J*_{gem} = 10.7, *J*_{5'a,4'} = 3.7, H-5'a); 3.757, 3.760 (2 × s, 2 × 3H, CH₃O-DMTr); 4.19 (ddd, 1H, *J*_{4',3'} = 4.1, *J*_{4'},_{5'} = 3.7, 2.9, H-4'); 4.46 (dd, 1H, *J*_{3',2'} = 5.0, *J*_{3',4'} = 4.1, H-3'); 4.72 (dd, 1H, *J*_{2',1'} = 5.4, *J*_{2',3'} = 5.0, H-2'); 6.37 (d, 1H, *J*_{1'2'} = 5.4, H-1'); 6.81 - 6.88 (m, 4H, H-*m*-C₆H₄-DMTr); 7.21 (m, 1H, H-*p*-C₆H₅-DMTr); 7.25 - 7.32 (m, 2H, H-*m*-C₆H₅-DMTr); 7.30 - 7.37 (m, 8H, H-*o*-C₆H₄-DMTr); 7.42 - 7.48 (m, 2H, H-*o*-C₆H₅-DMTr); 7.54 - 7.59 (m, 2H, H-m-Bz); 7.64 (m, 1H, H-*p*-Bz); 7.86 (s, 1H, H-6); 8.08 - 8.12 (m, 2H, H-o-Bz); 8.65 (s, 1H, H-2). ¹³C NMR (125.7 MHz, CD₃OD): 52.84 (C-5); 55.75, 55.76 (CH₃O-DMTr); 64.74 (CH₂-5'); 72.42 (CH-3'); 76.35 (CH-2'); 85.43 (CH-4'); 88.01 (C-DMTr); 89.41 (CH-1'); 114.23 (CH-*m*-C₆H₄-DMTr); 114.68 (C-4a); 127.99 (CH-*p*-C₆H₅-DMTr); 128.96 (CH-*m*-C₆H₅-DMTr); 129.23 (CH-o-Bz); 129.41 (CH-*o*-C₆H₅-DMTr); 129.90 (CH-m-Bz); 131.29, 131.40 (CH-*o*-C₆H₄-DMTr); 133.30 (CH-6); 133.78 (CH-*p*-Bz); 134.94 (C-*i*-Bz); 136.87, 137.15 (C-*i*-C₆H₄-DMTr); 146.04 (C-*i*-C₆H₅-DMTr); 152.24 (CH-2); 152.41 (C-4); 153.87 (C-7a); 160.17, 160.19 (C-*p*-C₆H₄-DMTr); 169.21 (C-*i*-Bz). ESI MS *m*/*z* (rel. %): 799 (23) [M+H]⁺, 821 (100) [M+Na]⁺, 837 (9) [M+K]+. HR MS (ESI) for C₃₉H₃₆O₇N₄I [M+H]⁺: calcd 799.16232; found 799.16154.

### Example 2

### 4-Benzamido-5-iodo-7-[2-O-tert-butyldimethylsilyl-5-O-(4,4'-dimethoxytrityl)-(β-_{D}-ribofuranosyl]-7H-pyrrolo[2,3-d]pyrimidine (4).

Nucleoside 3 (555 mg, 0.70 mmol) was co-evaporated with anhydrous pyridine (2× 5 ml) and dissolved in anhydrous THF (10 ml). Anhydrous pyridine (485 µl) and silver nitrate (195 mg, 1.15 mmol) were added. The reaction flask was covered with aluminum foil and the mixture was stirred at rt for 15 min. Then TBDMSC1 (183 mg, 1.21 mmol) was added and the mixture was stirred overnight in dark. Reaction mixture was then filtered through a pad of celite. The filtration pad was washed with DCM and the filtrate was evaporated. Flash chromatography on silica (5-50% EtOAc + 1% Et₃N in cyclohexane) provided nucleoside 4 (251 mg, 40 %) as a white foam. ¹H NMR (500.0 MHz, CD₃OD): -0.13, 0.01 (2 × s, 2 × 3H, CH₃Si); 0.81 (s, 9H, (CH₃)₃C); 3.43 (dd, 1H, *J*_{gem} = 10.7, J_{5'b,4'} = 3.0, H-5'b); 3.46 (dd, 1H, *J*_{gem} = 10.7, *J*_{5'a,4'} = 2.7, H-5'a); 3.779, 3.782 (2 × s, 2 × 3H, CH₃O-DMTr); 4.29 (ddd, 1H, *J*_{4',3'} = 3.1, *J*_{4',5'} = 3.0, 2.7, H-4'); 4.38 (dd, 1H, *J*_{3',2'} = 5.0, *J_{3',4'}* = 3.1, H-3'); 4.80 (dd, 1H, *J*_{2',1'} = 5.9, *J*_{2',3'} = 5.0, H-2'); 6.39 (d, 1H, *J*_{1',2'} = 5.9, H-1'); 6.85 - 6.90 (m, 4H, H-*m*-C₆H₄-DMTr); 7.24 (m, 1H, H-*p*-C₆H₅-DMTr); 7.28 - 7.33 (m, 2H, H-*m*-C₆H₅-DMTr); 7.34 - 7.39 (m, 8H, H-*o*-C₆H₄-DMTr); 7.45 - 7.49 (m, 2H, H-*o*-C₆H₅-DMTr); 7.55 - 7.60 (m, 2H, H*-m-*Bz); 7.66 (m, 1H, H-*p*-Bz); 7.91 (s, 1H, H-6); 8.09 - 8.13 (m, 2H, H-o-Bz); 8.65 (s, 1H, H-2). ¹³C NMR (125.7 MHz, CD₃OD): -4.94, -4.68 (CH₃Si); 18.99 ((CH₃)₃C); 26.15 ((CH₃)₃C); 53.05 (C-5); 55.80 (CH₃O-DMTr); 64.80 (CH₂-5'); 72.98 (CH-3'); 78.48 (CH-2'); 86.02 (CH-4'); 88.27 (C-DMTr); 89.24 (CH-1'); 114.32 (CH-*m*-C₆H₄-DMTr); 114.57 (C-4a); 128.11 (CH-*p*-C₆H₅-DMTr); 129.05 (CH-*m*-C₆H₅-DMTr); 129.23 (CH-o-Bz); 129.35 (CH-*o*-C₆H₅-DMTr); 129.93 (CH-m-Bz); 131.33, 131.43 (CH-*o*-C₆H₄-DMTr); 133.16 (CH-6); 133.82 (CH-p-Bz); 134.94 (C-*i*-Bz); 136.74, 136.99 (C-*i*-C₆H₄-DMTr); 145.99 (C-*i*-C₆H₅-DMTr); 152.29 (CH-2); 152.55 (C-4); 153.92 (C-7a); 160.25, 160.26 (C-*p*-C₆H₄-DMTr); 169.22 (C-*i*-Bz). ESI MS *m*/*z* (rel. %): 913 (100) [M+H]⁺. HR MS (ESI) for C₄₅H₅₀O₇N₄ISi [M+H]⁺: calcd 913.24880; found 913.24853.

### Example 3

### 4-Benzamido-5-iodo-7-[2-O-tert-butyldimethylsilyl-β-_{D}-ribofuranosyl]-7H-pyrrolo[2,3-d]pyrimidine 3'-O-phosphonate triethylammonium salt (5).

Nucleoside 4 (525 mg, 0.58 mmol) was co-evaporated with anhydrous pyridine (2× 5 ml) and dissolved in anhydrous pyridine (5 ml) and diphenyl phosphite (216 µl, 1.44 mmol) was added. The reaction mixture was stirred for 1 h and then water (8 ml) was added. The reaction mixture was stirred for 5 min and then it was diluted with EtOAc and washed with brine. Aqueous phase was extracted twice with EtOAc. Combined organic phases were dried over MgSO₄ and evaporated. The residue was dissolved in DCM (7 ml), water (104 µl) and solution of dichloroacetic acid in DCM (6%, 6.58 ml, 5.0 mmol) was added. The solution was stirred at rt for 15 min and then triethylsilane (919 µl, 5.75 mmol) was added and the reaction mixture was stirred for another 30 min. The reaction was quenched with pyridine (823 µl) and solvents were removed in vacuo. Flash chromatography on C18 column (gradient 5 - 100 % MeCN in 0.1M TEAB) provided phosphonate 12 (354 mg, 77 %) as a white foam. ¹H NMR (500.0 MHz, CD₃OD): -0.24, 0.01 (2 × s, 2 × 3H, CH₃Si); 0.77 (s, 9H, (CH₃)₃C); 1.31 (t, 9H, *J*_{vic} = 7.3, CH₃CH₂N); 3.20 (q, 6H, *J*_{vic} = 7.3, CH₃CH₂N); 3.87 (d, 2H, *J*_{5',4'} = 2.6, H-5'); 4.33 (q, 1H, *J*_{4',3'} =*J*_{4',5'} = 2.6, H-4'); 4.73 - 4.79 (m, 2H, H-2',3'); 6.33 (d, 1H, *J*_{*1*',2'} = 6.1, H-1'); 6.96 (d, 1H, *J*_{H,P} = 630.1, HP); 7.54 - 7.59 (m, 2H, H-m-Bz); 7.65 (m, 1H, H-*p*-Bz); 8.07 (s, 1H, H-6); 8.10 - 8.15 (m, 2H, H-o-Bz); 8.67 (s, 1H, H-2). ¹³C NMR (125.7 MHz, CD₃OD): -5.22, -4.60 (CH₃Si); 9.22 (CH₃CH₂N); 18.84 ((CH₃)₃C); 26.13 ((CH₃)₃C); 47.86 (CH₃CH₂N); 52.61 (C-5); 62.66 (CH₂-5'); 75.43 (d, *J*_{C,P} = 5.1, CH-3'); 77.09 (d, *J*_{C*,*P} = 3.0, CH-2'); 87.07 (d, *J*_{C,P} = 3.4, CH-4'); 89.77 (CH-1'); 114.84 (C-4a); 129.24 (CH-o-Bz); 129.89 (CH-m-Bz); 133.74 (CH-*p*-Bz); 133.93 (CH-6); 135.03 (C-*i*-Bz); 152.05 (CH-2); 152.68 (C-4); 153.65 (C-7a); 169.10 (C-*i*-Bz). ³¹P{¹H} NMR (202.4 MHz, CD₃OD): 5.24. ESI MS *m*/*z* (rel. %): 673 (100) [M-H]⁻. HR MS (ESI) for C₂₄H₃₁O₇N₄IPSi [M-H]: calcd 673.07498; found 673.07515.

### Example 4

### Cyclo-5-iodo-7-β-_{D}-ribofuranosyl-7H-pyrrolo[2,3-d]pyrimidine 5'-O-phosphate (3'→-5') guanosine 5'-O-phosphate (2'→-5') sodium salt (9).

Enzymatic cyclization: Nucleoside triphosphate 14 and GTP were enzymatically cyclized using mcGAS (General procedure A). The final product was purified by HPLC (5 - 30 % MeCN in 0.1M TEAB). The conversion to sodium salt form on Dowex 50WX8 (in Na⁺ cycle) provided cyclic dinucleotide 9 as a white lyophilizate (water).

Chemical synthesis: Phosphonate 5 (161 mg, 0.21 mmol) was dissolved in DCM (2.5 ml), water (38 µl, 2.11 mmol) and dichloroacetic acid in DCM (6%, 2.5 ml, 1.81 mmol) were added and the solution was stirred for 10 min, then pyridine (298 µl, 3.70 mmol) was added and the mixture was evaporated under reduced pressure and co-evaporated with anhydrous acetonitrile (3x). The residue was dissolved in anhydrous acetonitrile (480 µl) and a solution of phosphoramidite 6 (262 mg, 0.268 mmol) in anhydrous acetonitrile (1.45 ml) was added. The mixture was stirred at rt for 10 min and then *t*-butylhydroperoxide (5.5 M solution in decanol, 113 µl, 0.621 mmol) was added and the stirring continued for another 30 min. Then the solution was cooled to 0 °C and solution of NaHSO₃ (33%wt, 627 µl, 2.46 mmol) was added. The mixture was stirred for 10 min at 0 °C and then 5 min at rt Then solvent was removed in vacuo and the residue was dissolved in DCM (3.22 ml). Water (38 µl, 2.11 mmol) and dichloroacetic acid in DCM (6%, 3.22 ml, 2.33 mmol) were added. The mixture was stirred for 10 min, then pyridine (670 µl, 8.32 mmol) was added and the mixture was evaporated under reduced pressure and co-evaporated with water. Product 7 was partially purified by flash chromatography (C18 column, gradient 5 - 100% MeCN in 0.1 M TEAB).

Crude 7 was co-evaporated with anhydrous pyridine (3 × 2 ml) and dissolved in anhydrous pyridine (2.6 ml). DMOCP was added (85 mg, 0.46 mmol) and the mixture was stirred for 110 min at rt. Then, water (77 µl, 0.46 mmol) and iodine (44 mg, 0.46 mmol) were added and the stirring was continued for another 10 min at rt. The mixture was cooled to 0 °C and aqueous solution of NaHSO₃ (40%wt, 64 µl) was added. After stirring for 5 min at 0 °C, another portion of NaHSO₃ solution (40%wt, 40 µl) was added. The clear solution was evaporated under reduced pressure and co-evaporated with water. Crude 8 was partially purified by flash chromatography (C18 column, gradient 5 - 100% MeCN in 0.1M TEAB).

Crude 8 was dissolved in ethanolic solution of CH₃NH₂ (33%wt., 4 ml, 32.1 mmol) and the solution was stirred for 3 h at rt. Then the mixture was evaporated under reduced pressure and the residue was co-evaporated with anhydrous pyridine (3× 2ml). Mixture of anhydrous pyridine and Et₃N (1: 1v/v, 4 ml) and Et₃N.3HF (640 µl, 3.93 mmol) were added and the mixture was stirred at 50 °C for 3.5h. Then aqueous ammonium acetate (1M, 6 ml) was added and solvents were removed in vacuo. The residue was co-evaporated with water. After HPLC purification (0 ― 15 % MeCN in 0.1M TEAB) and conversion to sodium salt form on Dowex 50WX8 (in Na⁺ cycle) cyclic dinucleotide 9 (32 mg, 18% overall yield) was obtained as a white lyophilizate (water). ¹H NMR (600 MHz, D₂O): 4.14 (ddd, 1H, *J*_{5'b,4'} = 1.8, *J*_{5'b,5'a} = 11.7, *J*_{5'b,P} = 2.1, H5'b-G); 4.22 (ddd, 1H, *J*_{5'a,4'} = 3.1, *J*_{5'a,5'b} = 11.7, *J*_{5'a,P} = 4.6, H5'a-G); 4.23 (ddd, 1H, *J*_{5'b,5'a} = 12.0, *J*_{5'b,4'} = 1.3, *J*_{5'b,P} = 2.2, H5'b-A); 4.41 (ddd, 1H, *J*_{4',5'a} = 3.1, *J*_{4',5'b} = 1.8, *J*_{4',P} = 3.6, H4'-G); 4.44 (dm, 1H, *J*_{4',3'} = 9.3, H4'-A); 4.52 (dm, 1H, *J*_{5'a,5'b} = 12.0, H5'a-A); 4.61 (d, 1H, *J*_{3',2'} = 4.0, H3'-G); 4.67 (bd, 1H, *J*_{2',3'} = 4.0, H2'-A); 4.92 (ddd, 1H, *J*_{3',2'} = 4.0, *J*_{3',4'} = 9.3, *J*_{3'P} = 6.6, H3'-A); 5.62 (ddd, 1H, *J*_{2'*,*1'} = 8.6, *J*_{2',3'} = 4.0, *J*_{2'P} = 4.4, H2'-G); 5.94 (d, 1H, *J*_{1',2'} = 8.6, H1'-G); 6.10 (s, 1H, H1'-A); 7.71 (s, 1H, H6-A); 7.88 (s, 1H, H8-G); 8.09 (s, 1H, H2-A). ¹³C NMR (150.9 MHz, D₂O): 51.15 (C5-A); 65.23 (d, *J*_{C*,*P} = 4.5, C5'-A); 68.48 (d, *J*_{C,P}= 5.2, C5'-G); 72.88 (d, *J*_{C,P}= 5.6, C3'-A); 73.75 (C3'-G); 76.68 (C2'-A); 77.43 (d, *J*_{C*,*P} = 5.4, C2'-G); 82.55 (t, *J*_{C,P1} = *J*_{C,P2} = 11.3, C4'-A); 85.14 (d,*J*_{C,P} = 10.1, C4'-G); 89.10 (d,*J*_{C,P} = 12.0, C1'-G); 92.50 (C1'-A); 106.80 (C4a-A); 120.60 (C5-G); 129.42 (C6-A); 143.38 (C8-G); 150.09 (C7a-A); 153.95 (C2-A); 154.71 (C4-G); 155.84 (C2-G); 159.59 (C4'-A); 161.74 (C6-G). ³¹P NMR (¹H-dec, 202.4 MHz, D₂O): -0.32 and -1.41. ESI MS m/z (rel. %): 398 (100) [M-2H]²⁻, 798 (97) [M-H]⁻, 820 (30) [M-2H+Na]⁻. HR MS (ESI) for C₂₁H₂₃O₁₃N₉IP₂ [M-H]⁻: calcd 797.99407; found 797.99335.

### Example 5

### Cyclo-5-methyl-7-β-_{D}-ribofuranosyl-7H-pyrrolo[2,3-d]pyrimidine 5'-O-phosphate (3'→-5') guanosine 5'-O-phosphate (2'→-5') sodium salt (1a).

Nucleoside triphosphate **10** and GTP were enzymatically cyclized using mcGAS (General procedure A). The final product was purified by HPLC (5 - 30 % MeCN in 0.1M TEAB). The conversion to sodium salt form on Dowex 50WX8 (in Na⁺ cycle) provided cyclic dinucleotide **1a**. ¹H NMR (600 MHz, D₂O): 1.98 (d, 3H, *J*_{CH3,6} = 1.1, CH₃); 4.17 (m, 1H, H5'b-G); 4.18 (ddd, 1H, *J*_{5'b,5'a} = 11.7, *J*_{5'b,4'} = 2.4, *J*_{5'b,P} = 1.8, H5'b-A); 4.26 (ddd, 1H, *J*_{5'a,5'b} = 11.7, *J*_{5'a,4'} = 3.2, *J*_{3'P} = 4.7, H5'a-A); 4.43 (m, 2H, H4'-A and H4'-G); 4.44 (m, 1H, H5'a-G); 4.63 (d, 1H, *J*_{3',4'} = 4.1, H3'-G); 4.71 (bd, 1H, *J*_{2',3'} = 4.3, H2'-A); 5.06 (ddd, 1H, *J*_{3',2'} = 4.3, *J*_{3',4'} = 9.0, *J*_{3',P} = 6.7, H3'-A); 5.62 (ddd, 1H, *J*_{2'*,*1'} = 8.5, *J*_{2',3'} = 4.1, *J*_{2',P} = 6.6, H2'-G); 5.96 (d, 1H, *J*_{1',2'} = 8.5, H1'-G); 6.23 (s, 1H, H1'-A); 7.36 (q, 1H, *J*_{6,CH3} = 1.1, H6-A); 7.92 (s, 1H, H8-G); 8.19 (bs, 1H, H2-A). ¹³C NMR (150.9 MHz, D₂O; chemical shifts obtained from 2D-HSQC and 2D-HMBC spectra; J(C,P) and shifts of C4-A, C2-G and C6-G were not determined): 13.29 (CH₃); 64.98 (C5'-A); 68.33 (C5'-G); 73.32 (C3'-A); 73.85 (C3'-G); 77.08 (C2'-A); 77.49 (C2'-G); 82.64 (C4'-A); 86.11 (C4'-G); 88.85 (C1'-G); 92.20 (C1'-A); 105.17 (C4a-A); 114.97 (C5-A); 119.83 (C5-G); 123.61 (C6-A); 143.19 (C8-G); 147.84 (C2-A); 149.48 (C7a-A); 154.89 (C4-G). ³¹P NMR (¹H-dec, 202.4 MHz, D₂O): -0.27 and -1.18. ESI MS m/z (rel. %): 342 (100) [M-2H]²⁻, 353 (3) [M-3H+Na]²⁻, 686 (36) [M-H]⁻, 708 (8) [M-2H+Na]⁻. HR MS (ESI) for C₂₂H₂₇N₉O₁₃P₂: calcd 686.11308; found 686.11212.

### Example 6

### Cyclo-5-phenyl-7-β-_{D}-ribofuranosyl-7H-pyrrolo[2,3-d]pyrimidine 5'-O-phosphate (3'→-5') guanosine 5'-O-phosphate (2'→-5') sodium salt (1b).

Enzymatic synthesis: Nucleoside triphosphate 11 and GTP were enzymatically cyclized using mcGAS (General procedure A). The final product was purified by HPLC (5 - 30 % MeCN in 0.1 M TEAB). The conversion to sodium salt form on Dowex 50WX8 (in Na⁺ cycle) provided cyclic dinucleotide 1b.

Chemical synthesis: Cyclic dinucleotide 9 (15 mg, 17.8 µmol), phenylboronic acid (4.3 mg, 35.6 µmol) and cesium carbonate (17.4 mg, 53.4 µmol) were mixed with MeCN-H₂O (1:2v/v, 600 µl) in an argon purged vial. In a separate vial Pd(OAc)₂(1.0 mg, 4.45 µmol) and TPPTS (12.6 mg, 22.2 µmol) were dissolved in MeCN-H₂O (1:2v/v, 1.0 ml) and the solution was sonicated under argon atmosphere for 30s. Then 200 µmol (i.e. 1/5) of this solution was transferred into the mixture containing the cyclic dinucleotide 9 and the reaction was stirred at 100 °C for 30 min. Then the reaction mixture was cooled to rt, diluted with water to approx. 3 ml and filtered through a 5 µm nylon syringe filter. The filtrate was directly applied on HPLC for purification. After two HPLC (0 - 15 % MeCN in 0.1M TEAB) and conversion to sodium salt form on Dowex 50WX8 (in Na⁺ cycle) cyclic dinucleotide **1b** (2.5 mg, 18 %) was obtained as a white lyophilizate (water). ¹H NMR (600 MHz, D₂O): 4.15 (ddd, 1H, *J*_{5'b,4'} = 1.8, *J*_{5'b,5'a} = 11.8, *J*_{5'b,P} = 2.5, H5'b-G); 4.25 (ddd, 1H, *J*_{5'a,4'} = 2.8, *J*_{5'a,5'b} = 11.8, *J*_{5'a,P} = 5.0, H5'a-G); 4.29 (m, 1H, H5'b-A); 4.43 (ddd, 1H, *J*_{4',5'a} = 2.8, *J*_{4',5'b} = 1.8, *J*_{4',P} = 3.6, H4'-G); 4.49 (dm, 1H, *J*_{4',3'} = 9.1, H4'-A); 4.51 (m, 1H, H5'a-A); 4.66 (d, 1H, *J*_{3',2'} = 4.1, H3'-G); 4.79 (bd, 1H, *J*_{2',3'} = 4.1, H2'-A); 5.02 (ddd, 1H, *J*_{3',2'} = 4.1, *J*_{3',4'} = 9.1, *J*_{3',P} = 6.9, H3'-A); 5.73 (dt, 1H, *J*_{2',1'} = 8.6, *J*_{2',3'} = 4.1, *J*_{2',P} = 4.1, H2'-G); 6.01 (d, 1H, *J*_{1',2'} = 8.6, H1'-G); 6.29 (s, 1H, H1'-A); 7.09 (m, 2H, o-Ph-A); 7.24 (*m*, 2H, *m*-Ph-A); 7.25 (*m*, 1H, *p*-Ph); 7.63 (s, 1H, H6-A); 7.89 (s, 1H, H8-G); 8.22 (s, 1H, H2-A). ¹³C NMR (150.9 MHz, D₂O): 65.53 (d, *J*_{C,P}= 4.6, C5'-A); 68.66 (d, *J*_{C,P}= 5.4, C5'-G); 73.07 (d, *J*_{C,P}= 5.5, C3'-A); 73.93 (C3'-G); 76.96 (C2'-A); 77.87 (d, *J*_{C,P}= 4.6, C2'-G); 82.61 (t, *J*_{C,P} = 11.3, C4'-A); 86.04 (d, *J*_{C*,*P} = 9.7, C4'-G); 88.70 (d, *J*_{C,P}= 12.5, C1'-G); 92.27 (C1'-A); 103.79 (C4a-A); 119.40 (C5-A); 119.88 (C5-G); 122.74 (C6-A); 129.47 (*p*-Ph-A); 130.27 (o-Ph-A); 131.61 (*m*-Ph-A); 135.65 (*i*-Ph-A); 142.42 (C8-G); 151.35 (C7a-A); 153.88 (C2-A); 154.58 (C4-G); 155.99 (C2-G); 160.08 C4-A); 161.17 (C6-G). ³¹P NMR (¹H-dec, 202.4 MHz, D₂O): -0.19 and -0.90. ESI MS m/z (rel. %): 373 (100) [M-2H]²⁻, 385 (5) [M-3H+Na]²⁻, 770 (6) [M-H]⁻. HR MS (ESI) for C₂₇H₂₈O₁₃N₉P₂ [M-H]⁻: calcd 748.12873; found 748.12862.

### Example 7

### Cyclo-5-(naphthalen-2-yl)-7-β-_{D}-ribofuranosyl-7H-pyrrolo[2,3-d] pyrimidine 5'-O-phosphate (3'→-5') guanosine 5'-O-phosphate (2'→-5') sodium salt (1c)

Cyclic dinucleotide 1c was prepared from iodinated CDN 9 (13 mg, 15.4 µmol) and naphthalene-2-boronic acid (13.3 mg, 77.1 µmol) according to General procedure B. After HPLC purification (5 - 25 % MeCN in 0.1M TEAB), HPLC repurification (9 - 24 % MeCN in 0.1M TEAB) and conversion to sodium salt form on Dowex 50WX8 (in Na⁺ cycle) cyclic dinucleotide 1c (5.7 mg, 44%) was obtained as a white lyophilizate (water). ¹H NMR (600 MHz, D₂O): 4.13 (ddd, 1H, *J*_{5'b,4'} = 1.6, *J*_{5'b,5'a} = 11.8, *J*_{5'b,P} = 2.6, H5'b-G); 4.26 (ddd, 1H, *J*_{5'a,4'} = 2.7, *J*_{5'a,5'b} = 11.8, *J*_{5'a,P} = 4.5, H5'a-G); 4.36 (ddd, 1H, *J*_{5'b,4'} = 1.3, *J*_{5'b,5'a} = 12.1, *J*_{5'b,P} = 2.9, H5'b-A); 4.45 (ddd, 1H, *J*_{4',5'a} = 2.7, *J*_{4',5'b} = 1.6, *J*_{4',P} = 3.8, H4'-G); 4.53 (dm, 1H, *J*_{4',3'} = 9.6, H4'-A); 4.57 (ddd, 1H, *J*_{5'a,4'} = 2.3, *J*_{5'a,5'b} = 12.1, *J*_{5',P} = 1.1, H5'a-A); 4.73 (d, 1H, *J*_{3',4'} = 4.0, H3'-G); 4.81 (d, *J*_{3',2'} = 3.9, H2'-A); 5.03 (ddd, 1H, *J*_{3',2'} = 3.9, *J*_{3',4'} = 9.6, *J*_{3'P} = 6.6, H3'-A); 5.67 (um, 1H, H2'-G); 6.07 (d, 1H, *J*_{1',2'} = 8.6, H1'-G); 6.34 (s, 1H, H1'-A); 7.32 (dd, 1H, *J*_{3,2} = 1.8, *J*_{3,4} = 8.3, H3-naphth); 7.53 (m, 1H, H7-naphth); 7.545 (m, 1H, H6-naphth); 7.64 (dm, 1H, *J*_{1,3} = 1.8, H1- naphth); 7.74 (s, 1H, H8-G); 7.75 (s, 1H, H6-A); 7.77 (dm, 1H, *J*_{4,3} = 8.3, H4- naphth); 7.82 (m, 1H, H5- naphth); 7.90 (m, 1H, H8- naphth); 8.25 (s, 1H, H2-A). ¹³C NMR (150.9 MHz, D₂O): 65.66 (d, *J*_{C,P}= 4.7, C5'-A); 68.76 (d, *J*_{C,P}= 5.2, C5'-G); 73.16 (d, *J*_{C,P}= 5.4, C3'-A); 73.91 (C3'-G); 76.98 (C2'-A); 79.00 (C2'-G); 82.58 (t, *J*_{C,P}= 11.6, C4'-A); 86.13 (d, *J*_{C,P}= 9.6, C4'-G); 87.80 (C1'-G); 92.32 (C1'-A); 103.93 (C5-A); 119.40 (C4a-A); 119.61 (C5-G); 123.09 (C6-A); 128.19 (C1- naphth); 128.51 (C3- naphth); 128.70 (C7- naphth); 129.24 (C6- naphth); 130.26 (C8-naphth); 130.89 (C5- naphth); 131.18 (C4- naphth); 133.19 (C2- naphth); 134.43 (C4a- naphth); 135.96 (C8a-naphth); 141.25 (C8-G); 151.60 (C7a-A); 153.89 (C2-A); 154.34 (C4-G); 155.90 (C2-G); 160.32 (C4-A); 160.72 (C6-G). ³¹P NMR (¹H-dec, 202.4 MHz, D₂O): -0.12 and -0.96. ESI MS m/z (rel. %): 398 (100) [M-2H]²⁻, 798 (47) [M-H]⁻, 820 (35) [M-2H+Na]⁻ HR MS (ESI) for C₃₁H₂₉O₁₃N₉P₂ [M-2H]²⁻: calcd 398.56855; found 398.56823.

### Example 8

### Cyclo-5-(naphthalen-l-yl)-7β-_{D}-ribofuranosyl-7H-pyrrolo[2,3-d] pyrimidine 5'-O-phosphate (3'→-5') guanosine 5'-O-phosphate (2'→-5') sodium salt (1d)

Cyclic dinucleotide 1d was prepared according to General procedure B from iodinated cyclic dinucleotide 9 (15 mg, 17.8 µmol) and naphthalene-1-boronic acid (17.6 mg, 88.9 µmol). The final product was purified by HPLC (5 - 30 % MeCN in 0.1M TEAB). After HPLC repurification (5 - 50 % MeOH in 0.1M TEAB) and conversion to sodium salt form on Dowex 50WX8 (in Na⁺ cycle) cyclic dinucleotide **1d** (5.6 mg, 37%) was obtained as a white lyophilizate (water). The final product was a mixture of two diastereomers (63:37) due to hindered rotation (at 298.15 K). Chemical shifts of the resolved signals of minor isomer are given in italics. ¹H NMR (600 MHz, D₂O): 4.125 (dt, 1H, *J*_{5'b,4'} = 2.1, *J*_{5'b,5'a} = 11.8, *J*_{5'b,P} = 2.2, H5'b-G); *4.13* (ddd, 1H, *J*_{5'b},_{4'} = 2.3, *J*_{5'b,5'}. = 11.9, *J*_{5'b,P} = 3.5, H5'b-G); 4.23 (dd, 1H, *J*_{5'a,4'} = 2.6, *J*_{5'a,5'b} = 11.8, H5'a-G); 4.24 (dd, 1H, *J*_{5'a,4'} = 2.6, *J*_{5'a,5'b} = 11.9, H5'a-G); *4.25* (m, 1H, H5'b-A); 4.28 (ddd, 1H, *J*_{5'b,5'a} = 12.1, *J*_{5'b},_{4'} = 1.5, *J*_{5'b,P} = 2.8, H5'b-A); *4.385* (m, 1H, H4'-G); 4.39 (m, 1H, H4'-G); 4.44 (dm, 1H, *J*_{5'a,5'b} = 12.1, H5'a-A); *4.47* (dm, 1H, *J*_{4',3'} = 8.4, H4'-A); 4.50 (dm, 1H, *J*_{4',3'} = 9.0, H4'-A); 4.615 (d, 1H, *J*_{3',4'} = 4.1, H3'-G); *4.64* (d, 1H, *J*_{3',4'} = 4.1, H3'-G); 5.025 (d, 1H, *J*_{2',3'} = 4.3, H2'-A); *5.04* (dd, 1H, *J*_{2',3'} = 4.4, *J*_{2',1'} = 1.1, H2'-A); 5.09 (ddd, 1H, *J*_{3',2'} = 4.3, *J*_{3',4'} = 9.0, *J*_{3',P} = 6.2, H3'-A); *5.34* (ddd, 1H, *J*_{3',2'} = 4.4, *J*_{3',4'} = 8.4, *J*_{3',P} = 6.6, H3'-A); 5.79 (ddd, 1H, *J*_{2',1'} = 8.6, *J*_{2',3'} = 4.1, *J*_{2',P} = 3.7, H2'-G); *5.87* (dt, 1H, *J*_{2',1'} = 8.6, *J*_{2',3'} = 4.1, *J*_{2',P} = 3.9, H2'-G); 5.97 (d, 1H, *J*_{1',2'} = 8.6, H1'-G); *5.98* (d, 1H, *J*_{1',2'} = 8.6, H1'-G); 6.38 (s, 1H, H1'-A); *6.55* (d, 1H, *J*_{1',2'} = 1.1, H1'-A); 6.895 (dd, 1H, *J*_{2,3} = 7.0, *J*_{2,4} = 1.3, H8-naphth); *7.18* (dd, 1H, *J*_{3,4} = 8.3, *J*_{3,2} = 7.0, H3-naphth); *7.21* (s, 1H, H6-A); 7.36 (dd, 1H, *J*_{3,4} = 8.4, *J*_{3,2} = 7.0, H3-naphth); 7.38 (ddd, 1H, *J*_{6,7} = 8.2, *J*_{6,5} = 6.8, *J*_{6,8} = 1.5, H6-naphth); *7.38* (dd, 1H, *J*_{2,3} = 7.0, *J*_{2,4} = 1.5, H8-naphth); 7.41 (ddd, 1H, *J*_{7,8} = 6.8, *J*_{7,6} = 8.2, *J*_{7,5} = 1.4, H7-naphth); *7.485* (ddd, 1H, *J*_{6,7} = 8.5, *J*_{6,5} = 6.8, *J*_{6,8} = 1.3, H6-naphth); 7.52 (s, 1H, H6-A); *7.59* (ddd, 1H, *J*_{7,8} = 6.8, *J*_{7,6} = 8.5, *J*_{7,5} = 1.3, H7-naphth); *7.64* (dd, 1H, *J*_{5,6} = 6.8, *J*_{5,7} = 1.3, H5-naphth); 7.70 (dd, 1H, *J*_{5,6} = 6.8, *J*_{5,7} = 1.4, H5-naphth); *7.86* (dd, 1H, *J*_{4,3} = 8,3 *J*_{4,2} = 1.5, H4-naphth); 7.90 (dd, 1H, *J*_{8,7} = 6.8, *J*_{8,6} = 1.5, H8-naphth); 7.905 (dd, 1H, *J*_{4,3} = 8,4 *J*_{4,2} = 1.3, H4-naphth); 7.91 (s, 1H, H8-G); *7.995* (dd, 1H, *J*_{8,7} = 6.8, *J*_{8,6} = 1.3, H8-naphth); *8.20* (s, 1H, H2-A); 8.235 (s, 1H, H2-A). ¹³C NMR (150.9 MHz, D₂O): 65.48 (d, *J*_{C,P}= 4.5, C5'-A); *68.92* (d, *J*_{C,P} = 5.5, C5'-G); *65.98* (d, *J*_{C,P}= 5.0, C5'-A); 68.79 (d, *J*_{C,P} = 5.1, C5'-G); 73.59 (d, *J*_{C,P} = 5.4, C3'-A); *73.71* (d, *J*_{C,P}= 5.3, C3'-A); 74.19 (C3'-G); *74.32* (C3'-G); 76.70 (C2'-A); *76.84* (C2'-A); 77.34 (d, *J*_{C,P}= 5.2, C2'-G); *77.50* (d, *J*_{C,P}= 5.5, C2'-G); 82.55 (t, *J*_{C,P} = 11.3, C4'-A); *82.83* (t, *J*_{C,P} = 11.2, C4'-A); *85.72* (d, *J*_{C,P} = 9.1, C4'-G); 85.75 (d, *J*_{C,P} = 9.4, C4'-G); *88.63* (d, *J*_{C,P}= 12.5, C1'-G); 89.02 (d, *J*_{C,P} = 13.0, C1'-G); *90.32* (C1'-A); 91.67 (C1'-A); 105.81 (C4a-A); *106.86* (C4a-A); 117.04 (C5-A); *118.96* (C5-A); 119.75 (C5-G); *119.79* (C5-G); 123.68 (C6-A); *123.73* (C6-A); 127.65 (C5-naphth); 127.82 (C5-naphth); 128.11 (C3-naphth); 128.24 (C3-naphth); 128.92 (C7-naphth); *129.03* (C7-naphth); 129.64 (C6-naphth); 129.69 (C6-naphth); 130.82 (C2-naphth); 130.97 (C4-naphth); 131.06 (C8-naphth); 131.26 (C4-naphth); 131.45 (C2-naphth); 131.48 (C8-naphth); *132.48* (C4a-naphth); 132.73 (C4a-naphth); 134.11 (C8a-naphth); 134.28 (C8a-naphth); 136.12 (C1-naphth); *136.20* (C1-naphth); 142.91 (C8-G); *142.91* (C8-G); 151.38 (C7a-A); *152.67* (C7a-A); 154.21 (C2-A); 154.40 (C4-G); *154.40* (C4-G); *154.59* (C2-A); *156.04* (C2-G); 156.89 (C2-G); 159.90 (C4-A); *160.06* (C4-A); *160.28* (C6-G); 160.94 (C6-G). ³¹P NMR (¹H-dec, 202.4 MHz, D₂O): *-0.08*; -0.25; *-0.32*; -0.61. ESI MS m/z (rel. %): 398 (100) [M-2H]2⁻, 798 (25) [M-H]⁻. HR MS (ESI) for C₃₁H₃₀O₁₃N₉P₂ [M-H]⁻: calcd 798.114438; found 798.14472.

### Example 9

### Cyclo-5-(phenanthren-9-yl)-7-β-D-ribofuranosyl-7H-pyrrolo[2,3-d]pyrimidine 5'-O-phosphate (3'→5') guanosine 5'-O-phosphate (2'-→5') sodium salt (1e)

Cyclic dinucleotide **1e** was prepared according to General procedure B from iodinated cyclic dinucleotide 9 (15 mg, 17.8 µmol) and phenanthracene-9-boronic acid (19.4 mg, 88.9 µmol). The final product was purified by HPLC (5 - 30 % MeCN in 0.1M TEAB). The conversion to sodium salt form on Dowex 50WX8 (in Na⁺ cycle) provided cyclic dinucleotide **1e** (4.7 mg, 30%) as a white lyophilizate (water). The final product was a mixture of two diastereomers (63:37) due to hindered rotation (at 298.15 K). Chemical shifts of the resolved signals of minor isomer are given in italics. ¹H NMR (600 MHz, D₂O): 4.07 (dt, 1H, *J*_{5'b},_{4'} = 2.1, *J*_{5'b,5'a} = 11.8, *J*_{5'b,P} = 2.1, H5'b-G); *4.125* (dt, 1H, *J*_{5'b},_{4'} = 2.1, *J*_{5'b,5'a} = 11.8, *J*_{5'b,P} = 2.1, H5'b-G); 4.22 (ddd, 1H, *J*_{5'a,4'} = 2.4, *J*_{5'a,5'b} = 11.8, *J*_{5'a,P} = 5.1, H5'a-G); *4.25* (ddd, 1H, *J*_{5'a,4'} = 2.6, *J*_{5'a,5'b} = 11.8, *J*_{5'a,P} = 5.5, H5'a-G); *4.30* (ddd, 1H, *J*_{5'b,4'} = 1.6, *J*_{5'b,5'a} =11.8, *J*_{5'b,P} = 2.8, H5'b-A); 4.33 (m, 2H, H5'a-A and H5'b-A); 4.35 (dt, 1H, *J*_{4',5'a} = 2.4, *J*_{4',5'b} = 2.1, *J*_{4',P} = 3.6, H4'-G); *4.415* (dt, 1H, *J*_{4',5'a} = 2.6, *J*_{4',5'b} = 2.1, *J*_{4',P} = 3.4, H4'-G); *4.47* (dm, 1H, *J*_{5'a,5'b} = 11.8, H5'a-A); 4.49 (dm, 1H, *J*_{4',3'} = 8.6, H4'-A); *4.53* (dm, 1H, *J*_{4',3'} = 9.0, H4'-A); *4.66* (d, 1H, *J*_{3',2'} = 4.0, H3'-G); 4.67 (d, 1H, *J*_{3',2'} = 3.9, H3'-G); *5.09* (ddd, 1H, *J*_{3',2'} = 4.3, *J*_{3',4'} = 9.0, *J*_{3',P} = 6.3, H3'-A); 5.10 (d, 1H, *J*_{2',3'} = 4.5, H2'-A); 5.12 (d, 1H, *J*_{2',3'} = 4.3, H2'-A); 5.47 (ddd, 1H, *J*_{3',2'} = 4.5, *J*_{3',4'} = 8.6, *J*_{3',P} = 6.8, H3'-A); *5.80* (ddd, 1H, *J*_{2',1'} = 8.6, *J*_{2',3'} = 4.0, *J*_{2',P} = 3.5, H2'-G); 5.86 (d, 1H, *J*_{1',2'} = 8.6, H1'-G); 5.92 (um, 1H, H2'-G); *6.06* (d, 1H, *J*_{1',2'} = 8.6, H1'-G); *6.35* (s, 1H, H1'-A); 6.62 (s, 1H, H1'-A); *7.305* (s, 1H, H10-phen); 7.32 (s, 1H, H6-A); 7.34 (br, 1H, H8-G); *7.515* (ddd, 1H, *J*_{2,1} = 8.3, *J*_{2,3} = 6.9, *J*_{2,4} = 1.2, H2-phen); 7.61 (m,1H, H2-phen); *7.63* (m, 1H, H6-phen); 7.635 (m, 1H, H7-phen); *7.65* (s, 1H, H6-A); 7.725 (m, 2H, H6 and H8-phen); 7.75 (m, 1H, H1-phen); *7.765* (m, 1H, H3-phen); 7.77 (m, 1H, H7-phen); 7.785 (s, 1H, H10-phen); 7.79 (m, 1H, H3-phen); *7.805* (m, 1H, H1-phen); *7.90* (br, 1H, H8-G); 8.21 (s, 1H, H2-A); *8.25* (s, 1H, H2-A); 8.76 (dm, 1H, *J*_{4,3} = 8.5, H4-phen); *8.79* (dm, 1H, *J*_{5,6} = 8.5, H5-phen); 8.795 (dm, 1H, *J*_{4,3} = 8.5, H4-phen); 8.84 (dm, 1H, *J*_{5,6} = 8.5, H5-phen). ¹³C NMR (150.9 MHz, D₂O): *65.52* (C5'-A); 66.17 (C5'-A); *68.79* (C5'-G); 69.00 (C5'-G); *73.73* (C3'-A); 74.17 (C3'-G); 74.29 (C3'-G); 74.66 (C3'-A); *76.43* (C2'-A); 76.89 (C2'-A); 77.91 (C2'-G); *77.91* (C2'-G); *82.66* (C4'-A); 82.73 (C4'-A); 85.34 (C4'-G); *85.88* (C4'-G); 88.17 (C1'-G); *88.67*(C1'-G); 90.19 (C1'-A); *91.83* (C1'-A); 105.86 (C4a-A); *106.05* (C4a-A); *116.93* (C5-A); 118.78 (C5-G); 119.24 (C5-A); *119.48* (C5-G); *123.85* (C6-A); 124.16 (C6-A); *125.23* (C4-phen); 125.34 (C4-phen); *125.60* (C5-phen); 126.24 (C5-phen); 128.53 (C8-phen); 128.79 (C8-phen); *129.70* (C7-phen); 129.76 (C7-phen); 129.84 (C3-phen); 129.84 (C6-phen); *130.00* (C2-phen); 130.03 (C2-phen); 130.88 (C8a-phen); *131.18* (C10-phen); 131.48 (C10-phen); 131.49 (C8a-phen); 131.74 (C1-phen); *132.33* (C4a-phen); 132.35 (C1-phen); 132.45 (C4a-phen); *132.84* (C4b-phen); *133.02* (C9-phen);133.09 (C4b-phen); 133.18 (C9-phen); 141.58 (C8-G); *142.58* (C8-G); *151.23* (C7a-A); 152.86 (C7a-A); 153.68 (C4-G); *154.05* (C2-A); *154.37* (C4-G); 154.57 (C2-A); 155.96 (C2-G); *155.98* (C2-G); 159.90 (C4-A); 159.68 (C6-G); 159.74 (C6-G); *160.72* (C4-A). ³¹P NMR (¹H-dec, 202.4 MHz, D₂O): -0.07; *-0.23*; -0.44; *-0.68.* ESI MS m/z (rel. %): 423 (100) [M-2H]2⁻, 856 (9) [M-2H+Na]⁻ . HR MS (ESI) for C₃₅H₃₂O₁₃N₉P₂ [M-H]⁻: calcd 848.16003; found 848.15924.

### Example 10

### Cyclo-5-(biphenyl-4-yl)-7-β-D-ribofuranosyl-7H-pyrrolo[2,3-d]pyrimidine 5'-O-phosphate (3'→-5') guanosine 5'-O-phosphate (2'-→5') sodium salt (1f)

Cyclic dinucleotide If was prepared according to General procedure B from iodinated cyclic dinucleotide 9 (15 mg, 17.8 µmol) and 4-biphenylboronic acid (17.6 mg, 88.9 µmol). The final product was purified by HPLC (5 - 25 % MeCN in 0.1M TEAB). The conversion to sodium salt form on Dowex 50WX8 (in Na⁺ cycle) provided cyclic dinucleotide If (9.1 mg, 59 %) as a white lyophilizate (water). ¹H NMR (600 MHz, D₂O): 4.13 (ddd, 1H, *J*_{5'b},_{4'} = 1.7, *J*_{5'b,5'}. = 11.7, *J*_{5'b,P} = 2.5, H5'b-G); 4.24 (ddd, 1H, *J*_{5'a,a'} = 2.7, *J*_{5',5'b} = 11.7, *J*_{5'a,P} = 4.7, H5'a-G); 4.32 (ddd, 1H, *J*_{5'b,4'} = 2.9, *J*_{5'b,5'a} = 11.8, *J*_{5'b,P} = 1.2, H5'b-A); 4.42 (ddd, 1H, *J*_{4',5'a} = 2.7, *J*_{4',5'b} = 1.7, *J*_{4',P} = 3.7, H4'-G); 4.50 (dm, 1H, *J*_{4',3'} = 9.3, H4'-A); 4.56 (bdd, 1H, *J*_{5'a,4'} = 2.1, *J*_{5'a,5'b} = 11.8, *J*_{5'a,P} = <1, H5'a-A); 4.68 (d, 1H, *J*_{3',4'} = 4.1, H3'-G); 4.74 (d, 1H, *J*_{2',3'} = 4.0, H2'-A); 4.97 (ddd, 1H, *J*_{3',2'} = 4.0, *J*_{3',4'} = 9.3, *J*_{3',P} = 6.5, H3'-A); 5.64 (um, 1H, H2'-G); 6.02 (d, 1H, *J*_{1',2'} = 8.5, H1'-G); 6.25 (s, 1H, H1'-A); 7.11 (m, 2H, H3-phenylene + H5-phenylene); 7.41 (m, 1H, H4'-Ph); 7.48 (m, 2H, H2-phenylene + H6-phenylene); 7.51 (m, 2H, H3'-Ph + H5'-Ph); 7.66 (s, 1H, H6-A); 7.71 (m, 2H, H2'-Ph + H6'-Ph); 7.82 (s, 1H, H8-G); 8.18 (s, 1H, H2-A). ¹³C NMR (150.9 MHz, D₂O): 65.54 (d, *J*_{C,P} = 4.8, C5'-A); 68.72 (d, *J*_{C,P} = 5.2, C5'-G); 73.09 (d, *J*_{C,P} = 6.5, C3'-A); 73.80 (C3'-G); 76.97 (C2'-A); 78.50 (C2'-G); 82.55 (t, *J*_{C,P1} = *J*_{C,P2} = 11.3, C4'-A); 86.10 (d, *J*_{C,P} = 9.2, C4'-G); 88.07 (C1'-G); 92.24 (C1'-A); 103.65 (C4a-A); 119.08 (C5-A); 119.67 (C5-G); 122.79 (C6-A); 129.37 (C2'-Ph + C6'-Ph); 129.67 (C2-phenylene + C6-phenylene); 130.31 (C4'-Ph); 130.49 (C3-phenylene + C5-phenylene); 131.82 (C3'-Ph + C5'-Ph); 134.81 (C4-phenylene); 140.79 (C1-phenylene); 141.33 (C8-G); 142.49 (Cl'-Ph); 151.25 (C7a-A); 153.39 (C2-A); 154.49 (C4-G); 155.98 (C2-G); 159.95 (C4-A); 160.98 (C6-G). ³¹P NMR (¹H-dec, 202.4 MHz, D₂O): -0.14 and -0.93. ESI MS m/z (rel. %): 411 (100) [M-2M²⁻, 824 (43) [M-H]⁻, 846 (17) [M-2H+Na]⁻. HR MS (ESI) for C₃₃H₃₂O₁₃N₉P₂ [M-H]⁻: calcd 824.16003; found 824.16012.

### Example 11

### Cyclo-5-(4-(naphthalen-2-yl)phenyl)-7-β-D-ribofuranosyl-7H-pyrrolo[2,3-d]pyrimidine 5'-O-phosphate (3'→-5') guanosine 5'-O-phosphate (2'→5') sodium salt (1g)

Cyclic dinucleotide 1g was prepared according to General procedure B from iodinated cyclic dinucleotide 9 (15 mg, 17.8 µmol) and 4-(naphthalene-2-yl)phenylboronic acid pinacol ester (29.4 mg, 88.9 µmol). The final product was purified by HPLC (5 - 30 % MeCN in 0.1M TEAB). The conversion to sodium salt form on Dowex 50WX8 (in Na⁺ cycle) provided cyclic dinucleotide **1g** (5.3 mg, 32%) as a white lyophilizate (water). ¹H NMR (600 MHz, D₂O): 4.14 (ddd, 1H, *J*_{5'b},_{4'} = 1.8, *J*_{5'b,5'}. *=* 11.7, *J*_{5'b,P} = 2.4, H5'b-G); 4.22 (ddd, 1H, *J*_{5'a,4'} = 2.5, *J*_{5'a,5'b} = 11.7, *J*_{5'a,P} = 4.8, H5'a-G); 4.35 (ddd, 1H, *J*_{5'b,5'a} = 11.9, *J*_{5'b},_{4'} = 1.1, *J*_{5'b,P} = 2.7, H5'b-A); 4.44 (ddd, 1H, *J*_{4',5'a} = 2.5, *J*_{4',5'b} = 1.8, *J*_{4',P} = 3.7, H4'-G); 4.52 (dm, 1H, *J*_{4',3'} = 9.3, H4'-A); 4.58 (bdd, 1H,*J*_{5'a,5'b} = 11.9, *J*_{5',4'} = 2.0, *J*_{5'b,P} = <1, H5'a-A); 4.70 (d, 1H, *J*_{3',2'} = 4.0, H3'-G); 4.76 (overlap, H2'-A); 4.98 (ddd, 1H, *J*_{3',2'} = 4.0, *J*_{3',4'} = 9.3, *J*_{3',P} = 6.6, H3'-A); 5.61 (um, 1H, H2'-G); 6.03 (d, 1H, *J*_{1',2'} = 8.5, H1'-G); 6.30 (s, 1H, H1'-A); 7.16 (m, 2H, H2+H6-phenylene); 7.53 (m, 1H, H7-naphth); 7.54 (m, 1H, H6-naphth); 7.57 (m, 2H, H3+H5-phenylene); 7.65 (s, 1H, H6-A); 7.81 (s, 1H, H8-G); 7.84 (dd, 1H, *J*_{3,1} = 1.9, *J*_{3,4} = 8.6, H3-naphth); 7.90 (m, 1H, H8-naphth); 7.91 (m, 1H, H5-naphth); 7.94 (d, 1H, *J*_{4,3} = 8.6, H4-naphth); 8.12 (bd, 1H, *J*_{1,3} = 1.9, H1-naphth); 8.12 (s, 1H, H2-A). ¹³C NMR (150.9 MHz, D₂O): 65.71 (d, *J*_{C,P}= 3.2, C5'-A); 68.78 (d, *J*_{C,P} = 4.8, C5'-G); 73.19 (d, *J*_{C,P}= 6.5, C3'-A); 73.96 (C3'-G); 76.98 (C2'-A); 78.87 (C2'-G); 82.58 (t, *J*_{C,P1} = *J*_{C,P2} = 11.4, C4'-A); 86.12 (d, *J*_{C,P}= 9.5, C4'-G); 87.90 (d, *J*_{C,P}= 13.9, C1'-G); 92.18 (C1'-A); 103.64 (C4a-A); 119.16 (C5-A); 119.61 (C5-G); 122.71 (C6-A); 127.76 (C1-naphth); 127.81 (C3-naphth); 128.93 (C7-naphth); 129.24 (C6-naphth); 129.89 (C3 and C5-phenylene); 130.24 (C8-naphth); 130.57 (C2 and C6-phenylene); 130.82 (C5-naphth); 131.15 (C4-naphth); 134.88 (C1-phenylene); 134.99 (C4a-naphth); 136.05 (C8a-naphth); 139.85 (C2-naphth); 140.55 (C4-phenylene); 141.08 (C8-G); 151.23 (C7a-A); 153.51 (C2-A); 154.49 (C4-G); 156.00 (C2-G); 159.89 (C4-A); 160.98 (C6-G). ³¹P NMR (¹H-dec, 202.4 MHz, D₂O): -0.14 and -0.93. ESI MS m/z (rel. %): 436 (100) [M-2H]2⁻, 874 (17) [M-H]⁻, 896 (14) [M-2H+Na]⁻. HR MS (ESI) for C₃₇H₃₄O₁₃N₉P₂ [M-H]⁻: calcd 874.17568; found 874.17462.

### Example 12

### Cyclo-5-[4-{(naphthalen-1-yloxy)methyl}phenyl]-7-β-D-ribofuranosyl-7H-pyrrolo[2,3-d]pyrimidine 5'-O-phosphate (3'→5') guanosine 5'-O-phosphate (2'→5') sodium salt (1h)

Cyclic dinucleotide **1h** was prepared according to General procedure B from iodinated cyclic dinucleotide 9 (15 mg, 17.8 µmol) and [(1-naphthyloxy)methyl]phenylboronic acid (24.7 mg, 88.9 µmol). The final product was purified by HPLC (5 - 32.5 % MeCN in 0.1M TEAB). The conversion to sodium salt form on Dowex 50WX8 (in Na⁺ cycle) provided cyclic dinucleotide **1h** (5.7 mg, 34%) as a white lyophilizate (water). ¹H NMR (600 MHz, D₂O): 4.13 (dt, 1H, *J*_{5'b},_{4'} = 1.8, *J*_{5'b,5'a} = 11.8, *J*_{5'b,P} = 1.8, H5'b-G); 4.24 (ddd, 1H, *J*_{5'a,4'} = 2.7, *J*_{5'a,5'b} = 11.8, *J*_{5'a,P} = 4.8, H5'a-G); 4.30 (dm, 1H, *J*_{5'b,5'a} = 11.7, H5'b-A); 4.42 (m, 1H, H4'-G); 4.49 (dm, 1H, *J*_{4',3'} = 9.5, H4'-A); 4.53 (dm, 1H, *J*_{5'a,5'b} = 11.7, H5'a-A); 4.66 (d, 1H, *J*_{3',4'} = 4.0, H3'-G); 4.72 (bd, 1H, *J*_{2',3'} = 4.0, H2'-A); 4.97 (ddd, 1H, *J*_{3',2'} = 4.0, *J*_{3',4'} = 9.5, *J*_{3',P} = 6.5, H3'-A); 5.26 (d, 1H, *J*_{gem} = 11.5, O-CHₐH_{b}); 5.30 (d, 1H, *J*_{gem} = 11.5, O-CHₐH_{b}); 5.68 (dt, 1H, *J*_{2',1'} = 8.5, *J*_{2',3'} = 4.0, *J*_{2',P} = 4.0, H2'-G); 5.97 (d, 1H, *J*_{1',2'} = 8.5, H1'-G); 6.11 (s, 1H, H1'-A); 7.09 (bd, 1H, *J*_{2,3} = 7.6, H2-naphth-A); 7.12 (m, 2H, H2 and H6-phenylene); 7.34 (m, 2H, H3 and H5-phenylene); 7.47 (m, 2H, H3 and H7-naphth-A); 7.54 (s, 1H, H6-A); 7.54 (m, 2H, H4 and H6-naphth-A); 7.76 (s, 1H, H8-G); 7.87 (bd, 1H, *J*_{5,6} = 8.3, H5-naphthA); 8.03 (s, 1H, H2-A); 8.17 (bd, 1H, = 8.5, H8-naphth-A). ¹³C NMR (150.9 MHz, D₂O): 65.51 (d, *J*_{C,P} = 4.0, C5'-A); 68.67 (d, *J*_{C,P} = 5.1, C5'-G); 73.02 (CH₂O); 73.15 (d, *J*_{C,P}= 5.6, C3'-A); 73.91 (C3'-G); 76.88 (C2'-A); 78.06 (d, *J*_{C,P} = 5.8, C2'-G); 82.56 (t, *J*_{C,P} = 11.2, C4'-A); 85.95 (d, *J*_{C,P} = 9.4, C4'-G); 88.51 (d, *J*_{C,P} = 12.2, C1'-G); 92.09 (C1'-A); 103.57 (C4a-A); 109.29 (C2-naphth); 118.94 (C5-A); 119.81 (C5-G); 122.80 (C6-A); 123.49 (C4-naphth); 124.15 (C8-naphth); 127.72 (C4a-naphth); 128.35 (C7-naphth); 129.04 (C3-naphth); 129.41 (C6-naphth); 130.23 (C5-naphth); 130.46 (C2 and C6-phenylene); 130.98 (C3 and C5-phenylene); 136.61 (C1- phenylene); 136.86 (C8a-naphth-A); 137.84 (C4- phenylene); 142.06 (C8-G); 150.92 (C7a-A);153.37 (C2-A); 154.50 (C4-G); 155.92 (C2-G); 156.43 (C1-naphth-A); 159.61 (C4-A); 161.05 (C6-G). ³¹P NMR (¹H-dec, 202.4 MHz, D₂O): -0.21 and -0.96. ESI MS m/z (rel. %): 451 (100) [M-2H]²⁻, 462 (5) [M-3H+Na]²⁻, 904 (6) [M-H]⁻. HR MS (ESI) for C₃₈H₃₆O₁₄N₉P₂ [M-H]⁻: calcd 904.18624; found 904.18469.

### Example 13

### Cyclo-5-(furan-2-yl)-7-β-D-ribofuranosyl-7H-pyrrolo[2,3-d]pyrimidine 5'-O-phosphate (3'-5') guanosine 5'-O-phosphate (2'-→5') sodium salt (1i).

Nucleoside triphosphate **12** and GTP were enzymatically cyclized using mcGAS (General procedure A). The final product was purified by HPLC (5 - 30 % MeCN in 0.1M TEAB). The conversion to sodium salt form on Dowex 50WX8 (in Na⁺ cycle) provided cyclic dinucleotide **1i**. ¹H NMR (600 MHz, D₂O): 4.13 (ddd, 1H, *J*_{5'b},_{4'} = 1.9, *J*_{5'b,5'a} = 11.8, *J*_{5'b,P} = 2.0, H5'b-G); 4.23 (ddd, 1H, *J*_{5'a,4'} = 3.1, *J*_{5'a,5'b} = 11.8, *J*_{5'a,P} = 4.5, H5'a-G); 4.34 (ddd, 1H, *J*_{5'b,5'a} = 12.0, *J*_{5'b,4'} = 2.7, *J*_{5'b,P} = 1.1, H5'b-A); 4.42 (ddd, 1H, *J*_{4',5'a} = 3.1, *J*_{4',5'b} = 1.9, *J*_{4',P} = 3.6, H4'-G); 4.50 (dm, 1H, *J*_{4',3'} = 9.7, H4'-A); 4.61 (bdd, 1H, *J*_{5'a,5'b} = 12.0, *J*_{5'a,4'} = 2.2, *J*_{5'a,P} < 1.0, H5'a-A); 4.66 (d, 1H, *J*_{3',4'} = 4.0, H3'-G); 4.66 (d, 1H, *J*_{2',3'} = 4.0, H2'-A); 5.01 (ddd, 1H, *J*_{3',2'} = 4.0, *J*_{3',4'} = 9.7, *J*_{3',P} = 6.6, H3'-A); 5.74 (ddd, 1H, *J*_{2',1'} = 8.7, *J*_{2',3'} = 4.0, *J*_{2',P} = 3.8, H2'-G); 5.93 (d, 1H, *J*_{1',2'} = 8.7, H1'-G); 6.27 (s, 1H, H1'-A); 6.26 (dd, 1H, *J*_{4,3} = 3.5, *J*_{4,5} = 1.8, H4-furyl); 6.28 (dd, 1H, *J*_{3,4} = 3.5, *J*_{3,5} = 0.8, H3- furyl); 7.42 (dd, 1H, *J*_{5,4} = 1.8, *J*_{5,3} = 0.8, H5- furyl); 7.75 (s, 1H, H8-G); 8.00 (s, 1H, H6-A); 8.19 (s, 1H, H2-A). ¹³C NMR (150.9 MHz, D₂O): 65.48 (d, *J*_{C,P} = 4.0, C5'-A); 68.56 (d, *J*_{C,P}= 5.2, C5'-G); 72.66 (d, *J*_{C,P} = 5.3, C3'-A); 73.70 (C3'-G); 76.92 (C2'-A); 77.59 (br, C2'-G); 82.34 (t, *J*_{C,P1} = *J*_{C,P2} = 11.5, C4'-A); 85.82 (d, *J*_{C,P} = 10.2, C4'-G); 88.84 (d, *J*_{C,P} = 15.0, C1'-G); 92.50 (C1'-A); 102.40 (C5-A); 106.58 (C3-furyl); 109.14 (C4a-A); 114.75 (C4- furyl); 119.79 (C5-G); 120.95 (C6-A); 142.52 (C8-G); 143.61 (C5-furyl); 150.48 (C2- furyl);150.75 (C7a-A); 154.03 (C2-A); 154.47 (C4-G); 155.82 (C2-G); 159.59 (C4-A); 161.11 (C6-G). ³¹P NMR (¹H-dec, 202.4 MHz, D₂O): -0.16 and -1.18. ESI MS m/z (rel. %): 368 (100) [M-2H]²⁻, 379 (6) [M-3H-Na]²⁻, 738 (14) [M-H]⁻, 760 (14) [M-2H+Na]⁻. HR MS (ESI) for C₂₅H₂₆O₁₄N₉P₂ [M-H]⁻: cald 738.10799; found 738.10760.

### Example 14

### Cyclo-7-β-D-ribofuranosyl-5-(thiophen-2-yl)-7H-pyrrolo[2,3-d]pyrimidine 5'-O-phosphate (3'→5') guanosine 5'-O-phosphate (2'-→5') sodium salt (1j).

Nucleoside triphosphate 13 and GTP were enzymatically cyclized using mcGAS (General procedure A). The final product was purified by HPLC (5 - 30 % MeCN in 0.1M TEAB). The conversion to sodium salt form on Dowex 50WX8 (in Na⁺ cycle) provided cyclic dinucleotide **1i**. ¹H NMR (600 MHz, D₂O): 4.15 (ddd, 1H, *J*_{5'b},_{4'} = 1.8, *J*_{5'b,5'a} = 11.8, *J*_{5'b,P} = 2.2, H5'b-G); 4.29 (ddd, 1H, *J*_{5'a,4'} = 2.8, *J*_{5'a,5'b} = 11.8, *J*_{5'a,P} = 4.9, H5'a-G); 4.31 (ddd, 1H, *J*_{5'b,5'a} = 12.0, *J*_{5'b,4'} = 3.1, *J*_{5'b,P} = 1.2, H5'b-A); 4.44 (ddd, 1H, *J*_{4',5'a} = 2.8, *J*_{4',5'b} = 1.8, *J*_{4',P} = 3.6, H4'-G); 4.49 (dm, 1H, *J*_{4',3'} = 9.2, H4'-A); 4.52 (dm, 1H, *J*_{5'a,5'b} = 12.0, H5'a-A); 4.66 (d, 1H, *J*_{3',4'} = 4.0, H3'-G); 4.79 (overlap, H2'-A); 5.00 (ddd, 1H, *J*_{3',2'} = 4.2, *J*_{3',4'} = 9.2, *J*_{3',P} = 6.5, H3'-A); 5.70 (dt, 1H, *J*_{2',1'} = 8.7, *J*_{2',3'} = 4.0, *J*_{2',P} = 4.0, H2'-G); 5.99 (d, 1H, *J*_{1',2'} = 8.7, H1'-G); 6.28 (s, 1H, H1'-A); 6.82 (dd, 1H, *J*_{5,4} = 3.5, *J*_{5,3} = 1.2, H5-thienyl); 6.98 (dd, 1H, *J*_{4,3} = 5.2, *J*_{4,5} = 3.5, H4- thienyl); 7.24 (dd, 1H, *J*_{3,4} = 5.2, *J*_{3,5} = 1.2, H3-thienyl); 7.65 (s, 1H, H6-A); 7.87 (s, 1H, H8-G); 8.24 (s, 1H, H2-A). ¹³C NMR (150.9 MHz, D₂O): 65.53 (d, *J*_{C,P}= 4.2, C5'-A); 68.66 (d, *J*_{C,P}= 5.2, C5'-G); 73.03 (d, *J*_{C,P}= 5.6, C3'-A); 73.86 (C3'-G); 76.84 (C2'-A); 77.74 (C2'-G); 82.62 (t, *J*_{C,P}= 11.1, C4'-A); 85.96 (d, *J*_{C,P} = 9.8, C4'-G); 88.78 (d, *J*_{C,P} = 9.8, C1'-G); 92.20 (C1'-A); 103.70 (C5-A); 112.08 (C4a-A); 120.00 (C5-G); 123.16 (C6-A); 128.07 (C3-thienyl); 128.26 (C5- thienyl); 130.70 (C4- thienyl); 137.46 (C2- thienyl); 142.56 (C8-G); 151.16 (C7a-A); 154.12 (C2-A); 154.69 (C4-G); 155.97 (C2-G); 160.00 (C4-A); 161.29 (C6-G). ³¹P NMR (¹H-dec, 202.4 MHz, D₂O): -0.24 and -0.93. ESI MS m/z (rel. %): 376 (100) [M-2H]²⁻, 387 (4) [M-H+Na]²⁻ , 754 (4) [M-H]⁻, 776 (10) [M-2H+Na]⁻. HR MS (ESI) for C₂₅H₂₆O₁₃N₉P₂S [M-H]⁻: cald 754.08515; found 754.08502.

### Example 15

### Cyclo-5-(benzofuran-2-yl)-7-β-D-ribofuranosyl-7H-pyrrolo[2,3-d]pyrimidine 5'-O-phosphate (3'→-5') guanosine 5'-O-phosphate (2'-→5') sodium salt (1k)

Cyclic dinucleotide **1k** was prepared according to General procedure B from iodinated cyclic dinucleotide 9 (15 mg, 17.8 µmol) and benzofuran-2-ylboronic acid (14.4 mg, 88.9 µmol). After two purifications (5 - 25 % MeCN in 0.1M TEAB and 5 - 50 % MeOH in 0.1M TEAB) and conversion to sodium salt form on Dowex 50WX8 (in Na⁺ cycle) cyclic dinucleotide **1k** (8.1 mg, 55%) was obtained as a white lyophilizate (water). ¹H NMR (600 MHz, D₂O): 4.07 (ddd, 1H, *J*_{5'b},_{4'} = 1.8, *J*_{5'b,5'a} = 11.8, *J*_{5'b,P} = 2.4, H5'b-G); 4.20 (ddd, 1H, *J*_{5'a,4'} = 2.9, *J*_{5'a,5'b} = 11.8, *J*_{5'a,P} = 4.2, H5'a-G); 4.35 (ddd, 1H, *J*_{5'b},_{4'} = 1.0, *J*_{5'b,5'a} = 11.8, *J*_{5'b,P} = 2.6, H5'b-A); 4.38 (ddd, 1H, *J*_{4',5'a} = 2.9, *J*_{4',5'b} = 1.8, *J*_{4',P} = 3.6, H4'-G); 4.51 (dm, 1H, *J*_{4',3'} = 9.5, H4'-A); 4.59 (d, 1H, *J*_{2',3'} = 3.8, H2'-A); 4.65 (bdd, 1H, *J*_{5'a,4'} = 2.0, *J*_{5'a,5'b} = 11.8, *J*_{5'a,P} < 1, H5'a-A); 4.67 (d, 1H, *J*_{3',4'} = 3.8, H3'-G); 4.93 (ddd, 1H, *J*_{3',2'} = 3.8, *J*_{3',4'} = 9.5, *J*_{3',P} = 6.5, H3'-A); 5.62 (um, H2'-G); 5.90 (d, 1H, *J*_{1',2'} = 8.5, H1'-G); 6.18 (s, 1H, H1'-A); 6.65 (d, 1H, *J*_{3,4} = 3.8, H3-benzofuryl); 7.22 (td, 1H, *J*_{5,6} = 6.8, *J*_{5,4} = 6.8, *J*_{5,7} = 1.3, H5-benzofuryl); 7.24 (ddd, 1H, *J*_{6,5} = 6.8, *J*_{6,7} = 7.1, *J*_{6,4} = 1.6, H6-benzofuryl); 7.42 (m, 2H, H4-benzofuryl + H7-benzofuryl); 7.47 (s, 1H, H8-G); 8.09 (s, 1H, H2-A); 8.10 (s, 1H, H6-A). ¹³C NMR (150.9 MHz, D₂O): 65.53 (C5'-A); 68.59 (d, *J*_{C,P}= 3.7, C5'-G); 72.68 (d, *J*_{C,P}= 4.3, C3'-A); 73.74 (C3'-G); 78.30 (d, *J*_{C,P}= 5.4, C2'-G); 78.86 (C2'-A); 82.33 (t, *J*_{C,P1} = *J*_{C,P2} =10.6, C4'-A); 85.83 (d, *J*_{C,P}= 6.3, C4'-G); 88.02 (d, *J*_{C,P} = 12.0, C1'-G); 92.25 (C1'-A); 102.44 (C4a-A); 102.51 (C3-benzofuryl); 108.58 (C5-A); 113.48 (C7-benzofuryl); 119.31 (C5-G); 122.77 (C6-A); 123.46 (C4-benzofuryl); 125.99 (C5-benzofuryl); 126.32 (C6-benzofuryl); 131.49 (C3a-benzofuryl); 141.30 (C8-G); 150.77 (C7a-A); 152.57 (C2-benzofuryl); 153.71 (C2-A); 154.26 (C4-G); 155.77 (C2-G); 156.02 (C7a-benzofuryl); 159.60 (C4-A); 160.54 (C6-G). ³¹P NMR (¹H-dec, 202.4 MHz, D₂O): -0.10 and -1.10. ESI MS m/z (rel. %): 393 (100) [M-2H]²⁻, 788 (44) [M-H]⁻, 810 (21) [M-2H+Na]⁻. HR MS (ESI) for C₂₉H₂₈O₁₄N₉P₂ [M-H]⁻: calcd 788.12364; found 788.12413.

### Example 16

### Cyclo-5-(benzothiophen-2-yl)-7-β-D-ribofuranosyl-7H-pyrrolo[2,3-d]pyrimidine 5'-O-phosphate (3'→5') guanosine 5'-O-phosphate (2'-→5') sodium salt (11)

Cyclic dinucleotide 11 was prepared according to General procedure B from iodinated cyclic dinucleotide 9 (12 mg, 14.2 µmol) and benzothiophen-2-ylboronic acid (12.6 mg, 71.1 µmol). After two purifications (5 - 25 % MeCN in 0.1M TEAB and 9 - 24 % MeCN in 0.1M TEAB) and conversion to sodium salt form on Dowex 50WX8 (in Na⁺ cycle) cyclic dinucleotide 11 (4.8 mg, 40%) was obtained as a white lyophilizate (water). ¹H NMR (600 MHz, D₂O): 4.12 (ddd, 1H, *J*_{5'b,4'} = 1.6, *J*_{5'b,5'a} = 11.8, *J*_{5'b,P} = 2.5, H5'b-G); 4.25 (ddd, 1H, *J*_{5'a,4'} = 2.6, *J*_{5'a,5'b} = 11.8, *J*_{5'a,P} = 4.4, H5'a-G); 4.35 (ddd, 1H, *J*_{5'b},_{4'} ~ 1.0, *J*_{5'b,5'a} = 12.0, *J*_{5'b,P} = 2.5, H5'b-A); 4.44 (ddd, 1H, *J*_{4',5'a} = 2.6, *J*_{4',5'b} = 1.6, *J*_{4',P} = 3.8, H4'-G); 4.52 (dm, 1H, *J*_{4',3'} = 9.3, H4'-A); 4.57 (dm, 1H, *J*_{5'a,5'b} = 12.0, H5'a-A); 4.71 (d, 1H, *J*_{3',4'} = 4.0, H3'-G); 4.77 (overlap, H2'-A); 4.95 (ddd, 1H, *J*_{3',2'} = 4.0, *J*_{3',4'} = 9.3, *J*_{3',P} = 6.7, H3'-A); 5.60 (um, 1H, H2'-G); 6.03 (d, 1H, *J*_{1',2'} = 8.7, H1'-G); 6.30 (s, 1H, H1'-A); 7.10 (s, 1H, H3-benzothienyl); 7.36 (ddd, 1H, *J*_{6,4} = 1.3, *J*_{6,5} = 7.0, *J*_{6,7} = 8.0, H6-benzothienyl); 7.41 (ddd, 1H, *J*_{5,4} = 8.0, *J*_{5,6} = 7.0, *J*_{5,7} = 1.2, H5- benzothienyl); 7.72 (dd, 1H, *J*_{4,5} = 8.0, *J*_{4,6} = 1.3, H4- benzothienyl); 7.75 (s, 1H, H6-A); 7.78 (s, 1H, H8-G); 7.82 (dd, 1H, *J*_{7,6} = 8.0, *J*_{7,5} = 1.2, H7-benzothienyl); 8.23 (s, 1H, H2-A). ¹³C NMR (150.9 MHz, D₂O): 65.61 (b, C5'-A); 68.75 (d, *J*_{C,P} = 4.3, C5'-G); 72.96 (d, *J*_{C,P}= 5.4, C3'-A); 73.90 (C3'-G); 76.89 (C2'-A); 78.91 (C2'-G); 82.52 (t, *J*_{C,P} = 11.3, C4'-A); 86.08 (d, *J*_{C,P} = 9.6, C4'-G); 87.84 (C1'-G); 92.23 (C1'-A); 103.70 (C5-A); 112.55 (C4a-A); 119.66 (C5-G); 123.70 (C6-A); 124.04 (C3-benzothienyl); 124.89 (C7- benzothienyl); 126.22 (C4-benzothienyl); 126.99 (C6-benzothienyl); 127.37 (C6-benzothienyl); 141.14 (C8-G); 138.36 (C2-benzothienyl); 141.78 (C7a-benzothienyl); 142.94 (C3a-benzothienyl); 151.46 (C7a-A); 154.26 (C2-A); 154.59 (C4-G); 156.00 (C2-G); 160.18 (C4-A); 160.87 (C6-G). ³¹P NMR (¹H-dec, 202.4 MHz, D₂O): -0.16 and -0.88. ESI MS m/z (rel. %): 436 (100) [M-2H]²⁻, 874 (16) [M-H]⁻, 896 (14) [M-2H+Na]⁻. HR MS (ESI) for C₂₉H₂₈O₁₄N₉P₂ [M-H]⁻: calcd 874.17568; found 874.17462.

### Example 17

### Cyclo-5-(dibenzo[b,d]furan-4-yl)-7-β-_{D}-ribofuranosyl-7H-pyrrolo[2,3-d]pyrimidine 5'-O-phosphate (3'→-5') guanosine 5'-O-phosphate (2'-→5') sodium salt (1m)

Cyclic dinucleotide **1m** was prepared according to General procedure B from iodinated cyclic dinucleotide 9 (15 mg, 17.8 µmol) and dibenzofuran-4-ylboronic acid (19 mg, 89.6 µmol). After two purifications (5 - 25 % MeCN in 0.1M TEAB) and conversion to sodium salt form on Dowex 50WX8 (in Na⁺ cycle) cyclic dinucleotide **1m** (6.3 mg, 40 %) was obtained as a white lyophilizate (water). ¹H NMR (600 MHz, D₂O): 4.10 (ddd, 1H, *J*_{5'b,4'} = 2.1, *J*_{5'b,5'a} = 11.9, *J*_{5'b,P} = 2.4, H5'b-G); 4.22 (ddd, 1H, *J*_{5'a,4'} = 2.5, *J*_{5'a,5'b} = 11.9, *J*_{5'a,P} = 5.8, H5'a-G); 4.33 (m, 1H, H5'b-A); 4.38 (ddd, 1H, *J*_{4',5'a} = 2.5, *J*_{4',5'b} = 2.1, *J*_{4',P} = 3.4, H4'-G); 4.48 (m, 1H, H5'a-A); 4.49 (dm, 1H, *J*_{4',3'} = 9.0, H4'-A); 4.64 (d, 1H, *J*_{3',4'} = 3.5, H3'-G); 4.85 (d, 1H, *J*_{2',3'} = 4.6, H2'-A); 5.10 (ddd, 1H, *J*_{3',2'} = 4.6, *J*_{3',4'} = 9.0, *J*_{3',P} = 6.2, H3'-A); 5.84 (ddd, 1H, *J*_{2',1'} = 8.6, *J*_{2',3'} = 3.5, *J*_{2',P} = 3.7, H2'-G); 5.92 (d, 1H, *J*_{1',2'} = 8.6, H1'-G); 6.40 (s, 1H, H1'-A); 7.04 (dd, 1H, *J*_{3,2} = 7.5, *J*_{3,1} = 1.3, H3-dibenzofuryl); 7.14 (dd, 1H, *J*_{2,3} = 7.5, *J*_{2,1} = 7.8, H2-dibenzofuryl); 7.40 (td, 1H, *J*_{8,9} = 7.4, *J*_{8,7} = 7.4, *J*_{8,6} = 1.2, H8-dibenzofuryl); 7.44 (ddd, 1H, *J*_{7,6} = 8.2, *J*_{7,8} = 7.4, *J*_{7,9} = 1.5, H7-dibenzofuryl); 7.49 (s, 1H, H6-A); 7.59 (ddd, 1H, *J*_{6,7} = 8.2, *J*_{6,8} = 1.2, *J*_{6,9} = 0.7, H6-dibenzofuryl-A); 7.68 (s, 1H, H8-G); 7.76 (dd, 1H, *J*_{1,2} = 7.8, *J*_{1,3} = 1.3, H1-dibenzofuryl); 8.00 (ddd, 1H, *J*_{9,8} = 7.4, *J*_{9,7} = 1.5, *J*_{9,6} = 0.7, H9-dibenzofuryl); 8.19 (s, 1H, H2-A). ¹³C NMR (150.9 MHz, D₂O): 65.59 (d, *J*_{C,P} = 4.9, C5'-A); 68.80 (d, *J*_{C,P} = 4.9, C5'-G); 73.79 (d, *J*_{C,P} = 5.5, C3'-A); 74.22 (C3'-G); 77.14 (C2'-A); 77.50 (d, *J*_{C,P} =4.5, C2'-G); 82.84 (t, *J*_{C,P1} = *J*_{C,P2} = 11.2, C4'-A); 85.76 (d, *J*_{C,P} = 9.5, C4'-G); 88.79 (d, *J*_{C,P} = 12.3, C1'-G); 91.31 (C1'-A); 104.21 (C4a-A); 113.84 (C5-A); 114.61 (C6-dibenzofuryl); 119.57 (C4-dibenzofuryl); 119.60 (C5-G); 122.52 (C1-dibenzofuryl); 123.72 (C9-dibenzofuryl);124.05 (C6-A); 125.81 (C8-dibenzofuryl); 126.08 (C2-dibenzofuryl); 126.44 (C9a-dibenzofuryl); 127.01 (C9b-dibenzofuryl); 130.15 (C7-dibenzofuryl); 130.40 (C3-dibenzofuryl); 142.45 (C8-G); 151.79 (C7a-A); 154.22 (C4-G); 154.27 (C2-A); 155.08 (C4a-dibenzofuryl); 155.88 (C2-G); 158.29 (C5a-dibenzofuryl); 160.13 (C4-A); 160.59 (C6-G). ³¹P NMR (¹H-dec, 202.4 MHz, D₂O): -0.19 and -0.44. ESI MS m/z (rel. %): 418 (100) [M-2H]²⁻, 838 (28) [M-H]⁻, 860 (13) [M-2H+Na]⁻. HR MS (ESI) for C₃₃H₃₀O₁₄N₉P₂ [M-H]⁻: calcd 838.13929; found 838.13778.

### Biological activity screening

### Standard 293T Reporter Cell-Based Assay

The preparation of 293T reporter cell line stably expressing wild type STING protein and the assay itself was described previously (Novotná, B. et al. J. Med. Chem. 2019, 62, 10676-10690). Briefly, the day before testing cells were seeded onto 96-well white plates at density 250,000 cells per cm² in DMEM High glucose medium supplemented with 10 % (v/v) heat inactivated fetal bovine albumin (FBS). The next day medium was removed, and cell were incubated for 7 h (37 °C, 5 % CO₂) with serial dilution of compounds in medium. After incubation, Bright-Glo Luciferase Assay System reagent was added to cells and luminescence readout was performed on SPARK instrument (Tecan, Austria). EC₅₀ values were determined using in software Prism (GraphPad, USA).

### Peripheral Blood Mononuclear Cell (PBMC) Assay

Method was performed as described before in our previous work (Novotná, B. et al. J. Med. Chem. 2019, 62, 10676-10690). Briefly, buffy coats were obtained from the Institute of Hematology and Blood Transfusion (Prague, Czech Republic). Informed written consent was signed by each volunteer. Isolation of PBMCs was performed on Ficoll density gradient centrifugation (Ficoll Paque Plus, GE Healthcare, USA) in SepMate tubes (SepMate PBMC Isolation Tubes, Stemcell Technologies, Canada). Obtained PBMCs were washed using PBS with 2 mM EDTA, and then seeded onto 96-well U-shape plates at density 500,000 cells per well in RPMI 1640 media with 10 % (v/v) heat inactivated FBS. Serially diluted CDNs were added to cells and cells were incubated for 16 h (37 °C, 5 % CO₂). After incubation, medium was collected to determine levels of INFα and INFγ using ProcartaPlex Assays and MAGPIX System (Merck, Germany) according to the manufacturer's instructions. Cytotoxic effect of CDNs on PBMCs was determined using CellTiter-Glo^{®} Luminescent Cell Viability Assay (Promega Corporation, USA) according to manufacturer's instruction.

### Results of biological testing:

A cyclic dinucleotide was determined to be a STING agonist: (A) if it demonstrated STING activation through IRF-3 dependent expression of firefly luciferase reporter with EC₅₀ < 100 µmol.l⁻¹ in the standard 293T reporter cell-based assay and (B) if it showed induction of cytokines in PBMC assay with EC₅₀ < 100 µmol.l⁻¹. In the standard assay, **1a-1m** showed significantly improved activities compared with a reference compound In confirming that these derivatives are potent STING agonists. Compounds **1a-1m** showed significantly better activities in PBMC assay compared to In. All compounds induced INF γ and α with EC₅₀ values lower than that of In. Furthermore, **1a-1m** were not cytotoxic (CC₅₀ > 200 µmol.l⁻¹).

**Table 3: Biological activity of CDNs**

| Compound | Standard assay | | PBMC assay^{b} | |
|---|---|---|---|---|
| | EC₅₀ (µM)^{a} | EC₅₀ INF γ (µM) | EC₅₀ INF α (µM) | CC₅₀ (µM) |
| **1a** | 31.30 | 7.8 | 30.6 | >200 |
| **1b** | 40.7 | 6.84 | 7.36 | >200 |
| **1c** | 7.63 | 7.54 | 11.71 | >200 |
| **1d** | 42.45 | 34.51 | 28.78 | >200 |
| **1e** | 65.45 | 47.54 | 47.6 | >200 |
| **1f** | 2.95 | 2 | 7.4 | >200 |
| **1g** | 3.06 | 1.8 | 5.1 | >200 |
| **1h** | 2.58 | 1.08 | 1.97 | >200 |
| **1i** | 41.30 | 2.24 | 2.8 | >200 |
| **1j** | 27.30 | 2.6 | 2.88 | >200 |
| **1k** | 10.94 | 8.48 | 13.76 | >200 |
| **1l** | 8.69 | 47.54 | 47.6 | >200 |
| **1m** | 19.10 | 5.63 | 26.98 | >200 |
| **1n** | 66.4 | 67.6 | 54.5 | >200 |
| ^{a}Results of standard 293T reporter cell-based assay in 293T reporter cells expressing wt hSTING were obtained as described above. EC₅₀ values are the mean of three independent experiments (n=3) measured in triplicates with SD <50 % of EC₅₀ values; ^{b} Values are the mean from independent experiments obtained from three different PBMC donors. | | | | |

### Industrial Applicability

The compounds disclosed in this patent are useful as pharmaceuticals or components of drugs for treatment or prevention of a viral infection, hepatitis B virus infection, HIV infection, hyperproliferative disease or cancer, for enhancing the efficacy of vaccines and for modulating the activity of STING adaptor protein to induce production of type I interferon, cytokine and/or chemokine dependent on the STING adaptor protein, e.g., inducing a STING adaptor protein-dependent type I interferon, cytokine or chemokine in a human or an animal.

## Claims

1. 2',3'-Cyclic dinucleotide of general formula I: 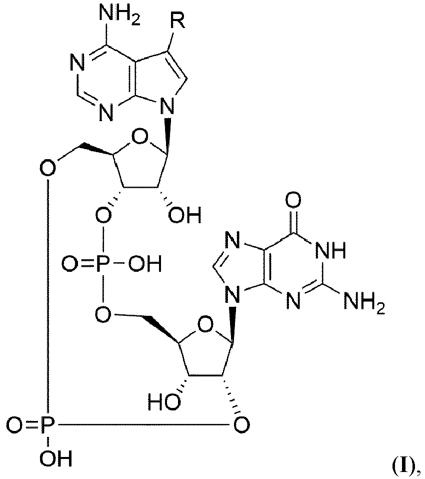 wherein
R is selected from the group comprising:
- C1-C5 alkyl;
- C6-C16 aryl, optionally substituted by at least one substituent selected from C6-C16 aryl or (C6-C16 aryloxy)C1-C5 alkyl;
- C4-C12 heteroaryl, comprising at least one O atom;
- C4-C12 heteroaryl, comprising at least one S atom,
or pharmaceutically acceptable salts thereof.

2. 2',3'-Cyclic dinucleotides of general formula I according to claim 1, wherein R is selected from the group comprising C1-C5 alkyl, phenyl, naphthalenyl, phenanthrenyl, furanyl, thiophenyl, benzofuranyl, benzothiophenyl, dibenzofuranyl, [1,1'-biphenyl]yl, (naphthalenyl)phenyl or [(naphthalenyloxy)methyl] phenyl.

3. 2',3'-Cyclic dinucleotide of general formula I according to claim 1, wherein R is selected from the group comprising methyl, phenyl, naphthalen-1-yl, naphthalen-2-yl, phenanthren-9-yl, furan-2-yl, thiophen-2-yl, benzo[*b*]furan-2-yl, benzo[*b*]thiophen-2-yl, dibenzo[*b*,*d*]furan-4-yl, [1,1'-biphenyl]-4-yl, 4-(naphthalenyl)phenyl and 4-[(naphthalen-1-yloxy)methyl]phenyl.

4. 2',3'-Cyclic dinucleotide of general formula I according to claim 1, selected from the following compounds:
2-amino-9-((5*R*,7*R*,8*R*,12a*R*,14*R*,15*R*,15a*S*,16*R*)-14-(4-amino-5-methyl-7*H*-pyrrolo[2,3-*d*]pyrimidin-7-yl)- 2,10,15,16-tetrahydroxy-2,10-dioxidooctahydro-12*H*-5,8-methanofuro[3,2-*l*][1,3,6,9,11]pentaoxa[2,10]diphosphacyclotetradecin-7-yl)-1,9-dihydro-6*H*-purin-6-one,
2-amino-9-((5*R*,7*R*,8*R*,12a*R*,14*R*,15*R*,15a*S*,16*R*)-14-(4-amino-5-phenyl-7*H*-pyrrolo[2,3-*d*]pyrimidin-7-yl)- 2,10,15,16-tetrahydroxy-2,10-dioxidooctahydro-12*H*-5,8-methanofuro[3,2-*l*][1,3,6,9,11]pentaoxa[2,10]diphosphacyclotetradecin-7-yl)-1,9-dihydro-6*H*-purin-6-one,
2-amino-9-((5*R*,7*R*,8*R*,12a*R*,14*R*,15*R*,15a*S*,16*R*)-14-(4-amino-5-(naphthalen-2-yl)-7*H*-pyrrolo[2,3-*d*]pyrimidin-7-yl)-2,10,15,16-tetrahydroxy-2,10-dioxidooctahydro-12*H*-5,8-methanofuro[3,2-*l*][1,3,6,9,11]pentaoxa[2,10]diphosphacyclotetradecin-7-yl)-1,9-dihydro-6*H*-purin-6-one,
2-amino-9-((5*R*,7*R*,8*R*,12a*R*,14*R*,15*R*,15a*S*,16*R*)-14-(4-amino-5-(naphthalen-1-yl)-7*H*-pyrrolo[2,3-*d*]pyrimidin-7-yl)-2,10,15,16-tetrahydroxy-2,10-dioxidooctahydro-12*H*-5,8-methanofuro[3,2-*l*][1,3,6,9,11]pentaoxa[2,10]diphosphacyclotetradecin-7-yl)-1,9-dihydro-6*H*-purin-6-one,
2-amino-9-((5*R*,7*R*,8*R*,12a*R*,14*R*,15*R*,15a*S*,16*R*)-14-(4-amino-5-(phenanthren-9-yl)-7*H*-pyrrolo[2,3-*d*]pyrimidin-7-yl)-2,10,15,16-tetrahydroxy-2,10-dioxidooctahydro-12*H*-5,8-methanofuro[3,2-*l*][1,3,6,9,11]pentaoxa[2,10]diphosphacyclotetradecin-7-yl)-1,9-dihydro-6*H*-purin-6-one,
9-((5*R*,7*R*,8*R*,12a*R*,14*R*,15*R*,15a*S*,16*R*)-14-(5-([1,1'-biphenyl]-4-yl)-4-arnino-7*H*-pyrrolo[2,3-*d*]pyrimidin-7-yl)-2,10,15,16-tetrahydroxy-2,10-dioxidooctahydro-12*H*-5,8-methanofuro[3,2-*l*][1,3,6,9,11]pentaoxa[2,10]diphosphacyclotetradecin-7-yl)-2-amino-1,9-dihydro-6*H*-purin-6-one,
2-amino-9-((5*R*,7*R*,8*R*,12a*R*,14*R*,15*R*,15a*S*,16*R*)-14-(4-amino-5-(4-(naphthalen-2-yl)phenyl)-7*H-*pyrrolo[2,3-*d*]pyrimidin-7-yl)-2,10,15,16-tetrahydroxy-2,10-dioxidooctahydro-12*H*-5,8-methanofuro[3,2-*l*][1,3,6,9,11]pentaoxa[2,10]diphosphacyclotetradecin-7-yl)-1,9-dihydro-6*H*-purin-6-one,
2-amino-9-((5*R*,7*R*,8*R*,12a*R*,14*R*,15*R*,15a*S*,16*R*)-14-(4-amino-5-(4-((naphthalen-1-yloxy)methyl)phenyl)-7*H*-pyrrolo[2,3-*d*]pyrimidin-7-yl)-2,10,15,16-tetrahydroxy-2,10-dioxidooctahydro-12*H*-5,8-methanofuro[3,2-*l*][1,3,6,9,11]pentaoxa[2,10]diphosphacyclotetradecin-7-yl)-1,9-dihydro-6*H*-purin-6-one,
2-amino-9-((5*R*,7*R*,8*R*,12a*R*,14*R*,15*R*,15a*S*,16*R*)-14-(4-amino-5-(furan-2-yl)-7*H*-pyrrolo[2,3-*d*]pyrimidin-7-yl)-2,10,15,16-tetrahydroxy-2,10-dioxidooctahydro-12*H*-5,8-methanofuro[3,2-*l*][1,3,6,9,11]pentaoxa[2,10]diphosphacyclotetradecin-7-yl)-1,9-dihydro-6*H*-purin-6-one,
2-amino-9-((5*R*,7*R*,8*R*,12a*R*,14*R*,15*R*,15a*S*,16*R*)-14-(4-amino-5-(thiophen-2-yl)-7*H*-pyrrolo[2,3-*d*]pyrimidin-7-yl)-2,10,15,16-tetrahydroxy-2,10-dioxidooctahydro-12*H*-5,8-methanofuro[3,2-*l*][1,3,6,9,11]pentaoxa[2,10]diphosphacyclotetradecin-7-yl)-1,9-dihydro-6*H*-purin-6-one,
2-amino-9-((5*R*,7*R*,8*R*,12a*R*,14*R*,15*R*,15a*S*,16*R*)-14-(4-amino-5-(benzo[*b*]furan-2-yl)-7*H*-pyrrolo[2,3-*d*]pyrimidin-7-yl)-2,10,15,16-tetrahydroxy-2,10-dioxidooctahydro-12*H*-5,8-methanofuro[3,2-*l*][1,3,6,9,11]pentaoxa[2,10]diphosphacyclotetradecin-7-yl)-1,9-dihydro-6*H*-purin-6-one,
2-amino-9-((5*R*,7*R*,8*R*,12a*R*,14*R*,15*R*,15a*S*,16*R*)-14-(4-amino-5-(benzo[*b*]thiophen-2-yl)-7*H*-pyrrolo[2,3-*d*]pyrimidin-7-yl)-2,10,15,16-tetrahydroxy-2,10-dioxidooctahydro-12*H*-5,8-methanofuro[3,2-*l*][1,3,6,9,11]pentaoxa[2,10]diphosphacyclotetradecin-7-yl)-1,9-dihydro-6*H*-purin-6-one,
2-amino-9-((5*R*,7*R*,8*R*,12a*R*,14*R*,15*R*,15a*S*,16*R*)-14-(4-amino-5-(dibenzo[*b*,*d*]furan-4-yl)-7*H*-pyrrolo[2,3-*d*]pyrimidin-7-yl)-2,10,15,16-tetrahydroxy-2,10-dioxidooctahydro-12*H*-5,8-methanofuro[3,2-*l*][1,3,6,9,11]pentaoxa[2,10]diphosphacyclotetradecin-7-yl)-1,9-dihydro-6*H*-purin-6-one,
or pharmaceutically acceptable salts thereof.

5. 2',3'-Cyclic dinucleotide of general formula I according to any one of claims 1 to 4 or a pharmaceutically acceptable salt thereof for use as a medicament.

6. 2',3'-Cyclic dinucleotide of general formula I according to any one of claims 1 to 4 or a pharmaceutically acceptable salt thereof for use in treatment and/or prevention of a viral infection, preferably hepatitis B virus infection and/or HIV infection.

7. 2',3'-Cyclic dinucleotide of general formula I according to any one of claims 1 to 4 or a pharmaceutically acceptable salt thereof for use in inhibition of pathological cell proliferation of tumor or non-tumor origin and/or in treatment of tumor or non-tumor disease associated with cell hyperproliferation.

8. 2',3'-Cyclic dinucleotide of general formula I according to any one of claims 1 to 4 or a pharmaceutically acceptable salt thereof for use as a vaccine adjuvans for enhancing efficacy of a vaccine.

9. 2',3'-Cyclic dinucleotide of general formula I according to any one of claims 1 to 4 or a pharmaceutically acceptable salt thereof for modulating the activity of STING adaptor protein to induce production of type I interferon, cytokine and/or chemokine dependent on the STING adaptor protein and/or for inducing a STING adaptor protein-dependent type I interferon, cytokine or chemokine in a human or an animal.

10. A pharmaceutical composition **characterized in that** it comprises a therapeutically effective amount of at least one compound of general formula I according to any of claims 1 to 4 or a pharmaceutically acceptable salt thereof, and at least one pharmaceutically acceptable carrier, filler and/or excipient.

11. The pharmaceutical composition according to claim 10 for use in inhibition of pathological cell proliferation of tumor or non-tumor origin and/or for treatment of tumor or non tumor disease associated with cell hyperproliferation.

12. The pharmaceutical composition according to claim 10 for use in treatment and/or prevention of a viral infection, preferably hepatitis B virus infection and HIV infection.

13. The pharmaceutical composition according to claim 10 for use in enhancing the efficacy of a vaccine.

14. The pharmaceutical composition according to claim 10 for use in modulating the activity of STING adaptor protein to induce production of type I interferon, cytokine and/or chemokine dependent on the STING adaptor protein, and/or for inducing a STING adaptor protein-dependent type I interferon, cytokine or chemokine in a human or an animal.
